(19) Europäisches Patentamt
European Patent Office
Office européen des brevets

(11) EP 3 076 938 B1

(12) # EUROPEAN PATENT SPECIFICATION

(45) Date of publication and mention of the grant of the patent:
**15.08.2018 Bulletin 2018/33**

(21) Application number: **14805271.5**

(22) Date of filing: **28.11.2014**

(51) Int Cl.:
***A61K 8/45*** (2006.01) ***A61K 8/37*** (2006.01)
***A61Q 3/02*** (2006.01) ***A61K 8/73*** (2006.01)

(86) International application number:
**PCT/EP2014/075919**

(87) International publication number:
**WO 2015/082335 (11.06.2015 Gazette 2015/23)**

(54) **PHOTO-CROSSLINKABLE VARNISH COMPOSITIONS AS BASE COATING AND APPLICATION METHODS**

LICHTVERNETZBARE LACKZUSAMMENSETZUNGEN ALS GRUNDSCHICHT UND ANWENDUNGSVERFAHREN

COMPOSITIONS PHOTORÉTICULABLES DE VERNIS COMME REVÊTEMENT DE BASE ET PROCÉDÉS D'APPLICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2013 FR 1362082**

(43) Date of publication of application:
**12.10.2016 Bulletin 2016/41**

(73) Proprietor: **L'OREAL**
**75008 Paris (FR)**

(72) Inventors:
- **KERGOSIEN, Guillaume**
  **F-92370 Chaville (FR)**
- **RIACHI, Carl**
  **F-75013 Paris (FR)**
- **LE PAPE, Marina**
  **94550 Chevilly-la-Rue (FR)**

(74) Representative: **Miszputen, Laurent**
**L'Oréal**
**Service DIPI**
**9 Rue Pierre Dreyfus**
**92110 Clichy (FR)**

(56) References cited:
**EP-A2- 0 453 628** **DE-A1-102011 102 661**
**GB-A- 2 496 990** **US-A1- 2011 081 306**
**US-A1- 2013 263 875**

- **Aniruddhans: "ESSTECH, Inc. - Product Catalog", , 7 June 2012 (2012-06-07), XP055076110, Retrieved from the Internet: URL:www.cenveomobile.com/i/69066/47 [retrieved on 2013-08-22]**
- **Esstech ET AL: "UV NAIL GEL PRODUCTS", , 10 May 2012 (2012-05-10), XP055075254, Retrieved from the Internet: URL:http://www.esstechinc.com/pdf/Esstech Nail Gel RM 020.pdf [retrieved on 2013-08-14]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 076 938 B1

## Description

The subject of the present invention is novel photo-crosslinkable varnish compositions. Compositions of this type preferably correspond to a base coating applied directly in contact with the nail and/or the false nail. This base coating can also be referred to as first coating in the case of a structure using a plurality of coatings of distinct compositions. This coating can thus be coated with at least one second coating. In particular, this second coating can be a top coating or a coloured coating. More particularly, the first coating can be coated with a coloured coating as second coating, which is itself coated with a top coating as third coating. The subject of the present invention is also methods for applying such compositions to nails and/or false nails, and also the use of said compositions for making up and/or caring for the nails and/or false nails.

The nail varnish compositions can be employed as a varnish base or base coat, as a product for making up the nails, or as a finishing composition, also known as a top coat, to be applied to the product for making up the nails, or else as a product for the cosmetic care of the nails. These compositions can be applied both to natural nails and to false nails.

In the nail varnish field, liquid cosmetic compositions are known which are used by first depositing a coating on the nail, and then by subjecting said coating to the action of light radiation, which causes *in situ* polymerisation and/or crosslinking reactions within said coating, resulting in polymeric networks which are usually crosslinked. Such photo-crosslinkable compositions, commonly known as "UV gels", and generally based on crosslinkable compounds of (meth)acrylate monomer type, make it possible to obtain a good wear property of the coating deposited on the nail, and are described, for example, in CA 1 306 954, US 5 456 905, US 7 375 144 and FR 2 823 105.

However, conventional "soak-off' UV gels generally exhibit wear property problems when they are not applied by expert manicurists. They generally also require a step of roughening the nail aimed at sanding down the nail in order to promote the wear property of the photo-crosslinked composition in film form, which can thus considerably damage the nail. Moreover, the removal of such compositions often proves to be difficult and can require a step of scraping the nail with a metal tool, an electric sander, or an abrasive file, harmful to the integrity of the nail.

Among the patent prior art aimed at overcoming these problems, mention may be made of documents US2011/0081306, US2011/0082228, US2011/0274633 and US2012/0083547.

Mention may be made also of the document US20130263875 disclosing compositions useful as an energy-curable nail enhancement requiring no base coat or top coat when applied to a nail, wherein the composition comprises at least one film-former and at least one energy-curable resin which may be selected from pyromellitic dianhydride di(meth)acrylate and pyromellitic di(meth)acrylate, pyromellitic dianhydride glyceryl dimethacrylate (PMGDM) and contains essentially no water or volatile non-reactive organic solvent. The cured composition exhibits good durability and yet can be readily and cleanly removed when desired by soaking briefly in acetone.

The present invention differs from this prior art through the development of a composition which has a wear property on the nails that is better than the competing products without roughening by sanding the nail, or with only slight roughening by sanding the nail, prior to the application of the photo-crosslinkable composition.

Furthermore, some products can exhibit performance problems regarding in particular the quality of the makeup result.

Moreover, the step of removing the prior art compositions conventionally uses tools intended to scrape the surface of the nail so as to remove the photo-crosslinked film of composition previously applied, which are capable of damaging the nails.

Finally, the present invention aims to provide novel photo-crosslinkable compositions which have, after photo-crosslinking of the film, a low content of extractable compounds comprising reactive (meth)acrylate functions.

The present invention thus aims to provide novel photo-crosslinkable compositions which do not exhibit at least one of the drawbacks of the compositions mentioned above.

In particular, the present invention aims to provide photo-crosslinkable compositions which can be removed with conventional organic solvents, such as acetone, without requiring a tool which is abrasive for the nails.

In particular, the present invention aims to provide photo-crosslinkable compositions which exhibit a good compromise between wear property and makeup removal compared with the photo-crosslinkable compositions described in the prior art or which exist.

The present invention also aims to provide photo-crosslinkable compositions which allow a quality nail makeup result, in particular in terms of homogeneity of result and, where appropriate, of colour.

The present invention aims to provide photo-crosslinkable compositions which are easy to use, including by the user herself, thus making it possible to save time and money.

The present invention relates to a photo-crosslinkable cosmetic composition in particular for coating a nail, and more particularly for making up a nail, comprising, in a physiologically acceptable medium:

- at least one photo-crosslinkable compound a) comprising at least two (ALK)acrylate functions and at least one

carboxylic acid function **and corresponding to formula (I-1) below:**

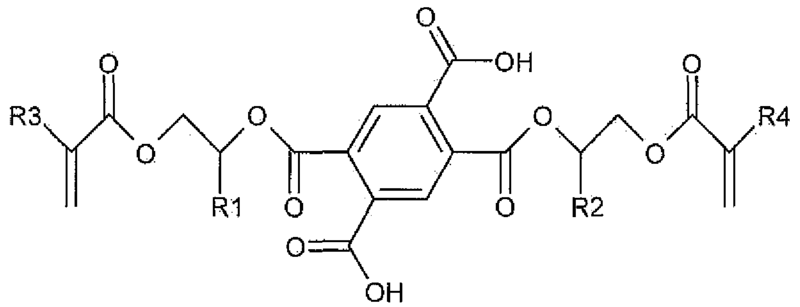

**(I-1)**

**in which formula (I-1):**

- **R3 and R4, which may be identical or different, represent a hydrogen atom or a methyl group ;**
- **R1 and R2, which may be identical or different, represent a hydrogen atom, a methyl group or a group of formula (II-1) below:**

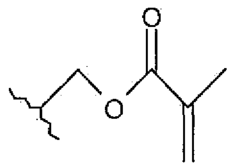

**(II-1) ;**

- at least one photo-crosslinkable compound b) **corresponding to formula (XI) below:**

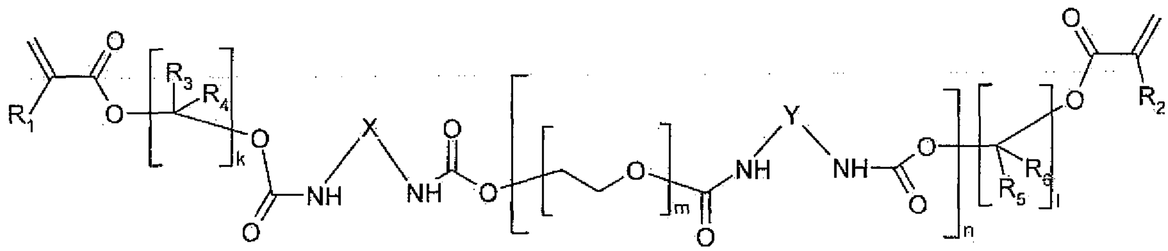

**Formula (XI)**

in which formula (XI):

- $R_1$, $R_2$, $R_3$, $R_4$, $R_5$, and $R_6$, which may be identical or different, represent a hydrogen atom or a $C_1$-$C_{10}$ alkyl chain, preferably a hydrogen atom or a methyl group,
- k and l, which may be identical or different, are between 1 and 10, preferably equal to 2,
- m is between 1 and 100, preferably between 5 and 50, preferably between approximately 8 and 10,
- n is between 1 and 10, preferably equal to 1,
- X and Y, which may be identical or different, represent a $C_1$-$C_{20}$ alkyl or cycloalkyl group,
- at least one film-forming polymer present in a total content strictly less than 30% by weight, relative to the total weight of the solids of the composition.

According to one preferred embodiment

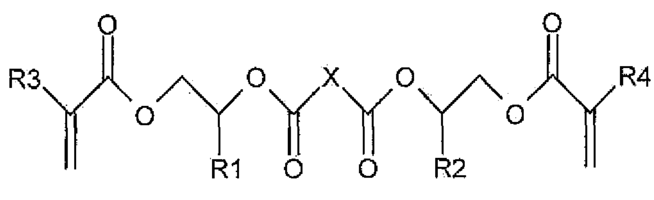

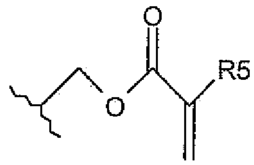

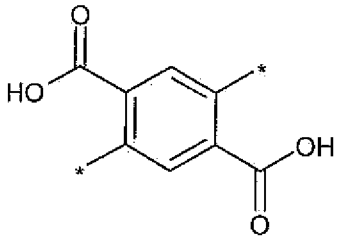

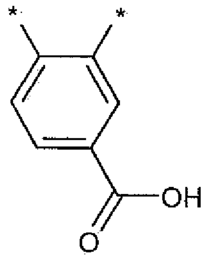

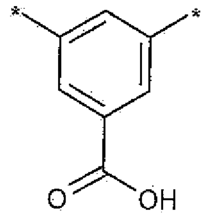

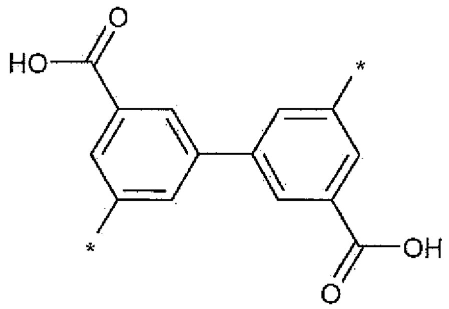

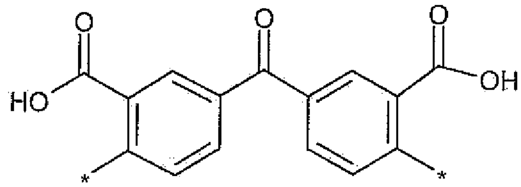

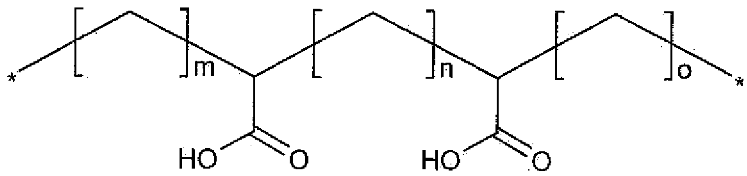

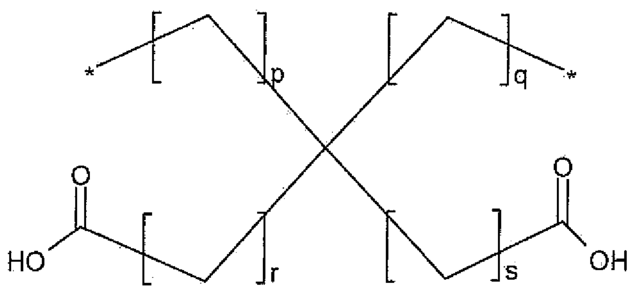

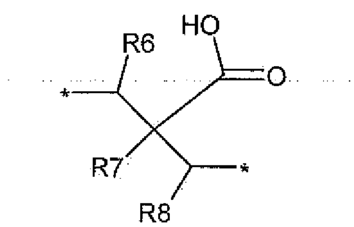

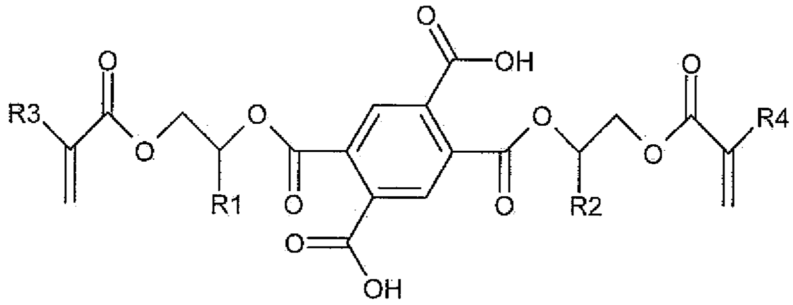

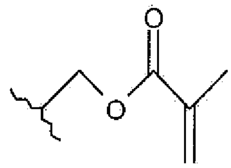

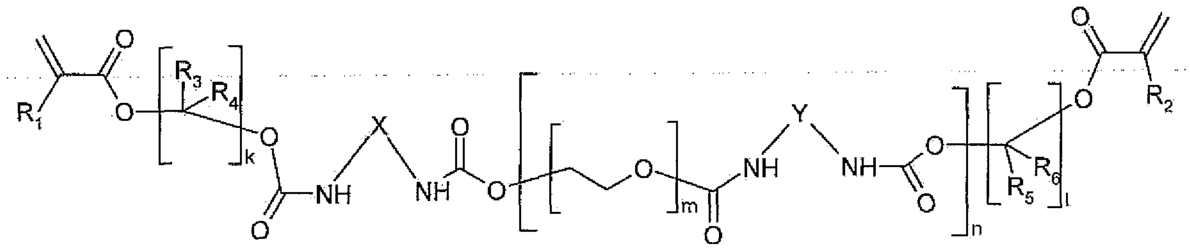

- the compound(s) b) corresponding to formula (XI), is (are) present in a total content greater than or equal to 20% by weight relative to the total weight of solids of the composition, in particular between 25% and 50% by weight and more particularly between 30% and 50% by weight, relative to the total weight of solids of the composition; the film-forming polymer(s) is (are) present in a total content greater than or equal to 20% by weight, relative to the total weight of solids of the composition, preferably ranging from 25% to 30% by weight, relative to the total weight of solids of the composition;
- the composition comprises at least one film-forming polymer chosen from the group consisting of poly(meth)acrylates, polysaccharides and derivatives, and a mixture thereof, preferably a mixture thereof;
- the film-forming polymer(s) comprise(s) at least one poly(meth)acrylate corresponding to formula (XII) below:

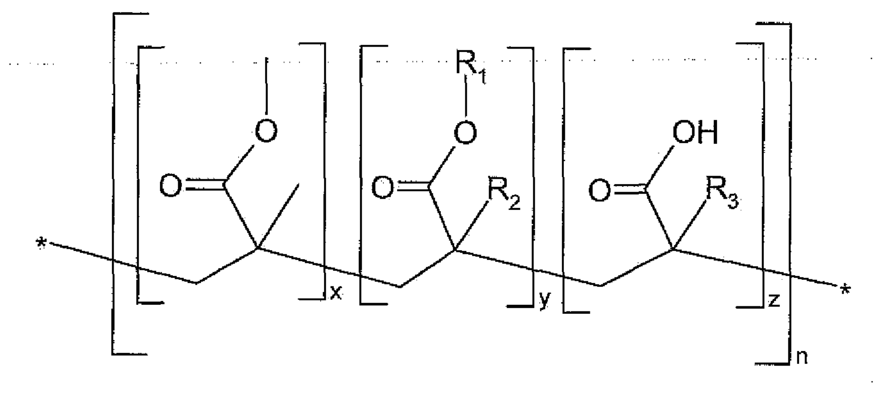

Formula (XII)

in which formula (XII):

- $R_1$, $R_2$ and $R_3$, which may be identical or different, represent a hydrogen atom or a $C_1$-$C_{10}$ alkyl group, $R_1$ preferably representing a $C_4$-$C_{10}$ alkyl group, and $R_2$ and $R_3$ preferably representing a hydrogen atom or a methyl group,
- x and y, which may be identical or different, represent an integer between 1 and 100,

- z represents an integer between 0 and 100,
- n represents an integer between 1 and 1000;

- the film-forming polymer(s), in particular chosen from the group consisting of poly(meth)acrylates, in particular corresponding to formula (XII), is (are) present in a total content greater than or equal to 10% by weight, relative to the total weight of solids of the composition, in particular a content ranging from 13% to 20% by weight, relative to the total weight of solids of the composition;
- the film-forming polymer(s) comprise(s) at least one polysaccharide or polysaccharide derivative chosen from nitrocellulose and ethers and esters of polysaccharides, in particular of $C_2$-$C_4$, in particular from cellulose acetobutyrates, cellulose acetopropionates, ethylcelluloses, ethyl guars, and mixtures thereof, more preferentially chosen from nitrocellulose;
- the film-forming polymer(s), in particular chosen from the group consisting of polysaccharides or polysaccharide derivatives is (are) present in a total content greater than or equal to 5% by weight, relative to the total weight of solids of the composition, in particular a content ranging from 10% to 15% by weight, relative to the total solids of the composition;
- the composition comprises at least one volatile solvent, preferably at least one polar volatile solvent, advantageously chosen from the group consisting of $C_3$-$C_6$ esters and ketones and mixtures thereof, preferably present in a total content greater than or equal to 30% by weight, relative to the total weight of the composition, in particular ranging from 50% to 70% relative to the total weight of the composition;
- the composition comprises at least one photoinitiator, the photoinitiator preferably being chosen from the group consisting of $\alpha$-hydroxy ketones, $\alpha$-amino ketones, aromatic ketones preferably combined with a hydrogen-donating compound, aromatic $\alpha$-diketones and acylphosphine oxides, and mixtures thereof, advantageously from the group consisting of acylphosphine oxides;
- the composition also comprises at least one (meth)acrylate monomer, preferably distinct from the compounds a), b), preferably in a content less than or equal to 10% by weight, relative to the total weight of the solids of the composition, better still less than or equal to 5% by weight, relative to the total weight of the solids of the composition;
- the composition is transparent.

The present invention also relates, according to a second aspect of the invention, to a method for coating the nails and/or false nails, in particular for making up and/or caring for the nails and/or false nails, comprising at least the following steps:

A) application, to a nail or a false nail, of a composition as previously defined, via which a coating consisting of at least one coat of said photo-crosslinkable composition is deposited, this coating being applied directly in contact with the nail or the false nail, and
B) exposure of the coated nail or false nail obtained at the end of step A) to a UV or visible light radiation.

The present invention relates more particularly to a method for coating the nails and/or false nails, in particular for making up and/or caring for the nails and/or false nails, comprising at least the following steps:

A) application, to a nail or a false nail, of a first composition as previously defined, via which a first coating consisting of at least one coat of said photo-crosslinkable composition is deposited, this first coating being applied directly in contact with the nail or the false nail,
B) exposure of the coated nail or false nail obtained at the end of step A) to a UV or visible light radiation,
C) application, to the first coating resulting from step A) and B), of a second composition, distinct from the first composition, via which a second coating consisting of at least one coat of said second composition is deposited,
D) exposure of the coated nail or false nail obtained at the end of step C) to a UV or visible light radiation.

In such a method, the second coating is preferably a top coat, optionally free of colouring agent.
The present invention also relates more particularly to a method for coating the nails and/or false nails, in particular for making up and/or caring for the nails and/or false nails, comprising at least the following steps:

A) application, to a nail or a false nail, of a first composition as previously defined, via which a first coating consisting of at least one coat of said photo-crosslinkable composition is deposited, this first coating being applied directly in contact with the nail or the false nail, and
B) exposure of the coated nail or false nail obtained at the end of step A) to a UV or visible light radiation,
C) application, to the first coating resulting from step A) and B), of a second composition, distinct from the first composition, via which a second coating consisting of at least one coat of said second composition is deposited,

D) exposure of the coated nail or false nail obtained at the end of step C) to a UV or visible light radiation,
E) application, to the second coating resulting from step C) and D), of a third composition, distinct from the first composition and from the second composition, via which a third coating consisting of at least one coat of said third composition is deposited,
F) exposure of the coated nail or false nail obtained at the end of step E) to a UV or visible light radiation.

The present invention relates more particularly to a method for coating the nails and/or false nails, in particular for making up and/or caring for the nails and/or false nails, comprising at least the following steps:

A) application, to a nail or a false nail, of a first composition as previously defined, via which a first coating consisting of at least one coat of said photo-crosslinkabie composition is deposited,
B) exposure of the coated nail or false nail obtained at the end of step A) to a UV or visible light radiation,
C) application, to the first coating resulting from step A) and B), of a second composition, distinct from the first composition, via which a second coating consisting of at least one coat of said second composition is deposited, the second composition comprising:

- at least one urethane (meth)acrylate compound corresponding to formula (XIII) below:

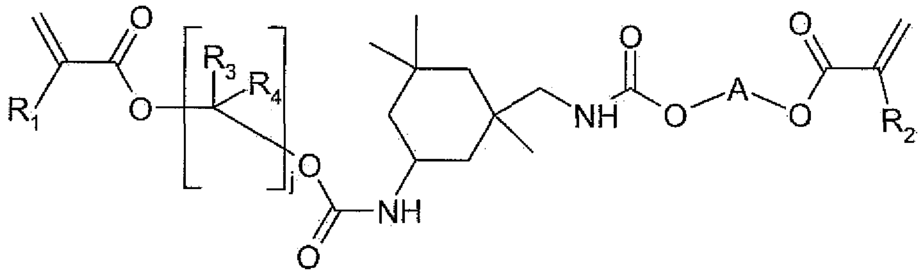

Formula (XIII)

in which formula XIII:

- $R_1$, $R_2$, $R_3$ and $R_4$, which may be identical or different, represent a hydrogen atom or a $C_1$-$C_{10}$ alkyl chain, preferably a hydrogen atom or a methyl group,
- j is between 1 and 10, preferably equal to 2,
- A represents a $C_1$-$C_{10}$ alkyl group, or a polyurethane comprising from 2 to 20 carbamate units,

- at least one photo-crosslinkable compound b) corresponding to formula (XI) below:

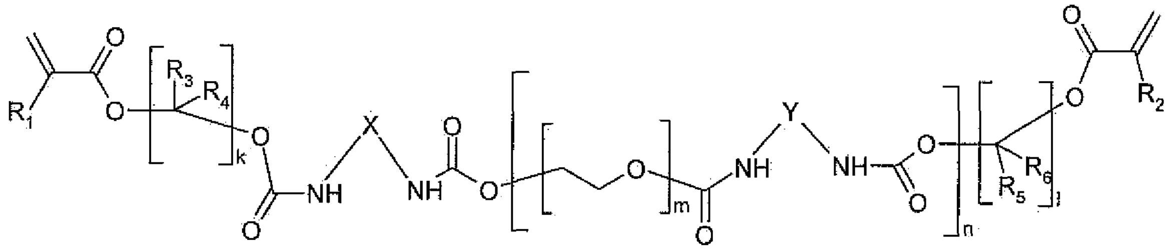

Formula (XI)

in which formula (XI):

- $R_1$, $R_2$, $R_6$, $R_4$, $R_6$, and $R_6$, which may be identical or different, represent a hydrogen atom or a $C_1$-$C_{10}$ alkyl chain, preferably a hydrogen atom or a methyl group,
- k and l, which may be identical or different, are between 1 and 10, preferably equal to 2,
- m is between 1 and 100, preferably between 5 and 50, preferably between approximately 8 and 10,
- n is between 1 and 10, preferably equal to 1,
- X and Y, which may be identical or different, represent a $C_1$-$C_{20}$ alkyl or cycloalkyl group,

- at least one photoinitiator, preferably chosen from the group consisting of α-hydroxy ketones, α-amino ketones, aromatic ketones preferably combined with a hydrogen-donating compound, aromatic α-diketones and acylphosphine oxides, and mixtures thereof, advantageously from the group consisting of acylphosphine oxides,
- preferably at least one monofunctional (meth)acrylate monomer, preferably tetrahydrofurfuryl methacrylate (THFMA),
- preferably at least one film-forming polymer,
- optionally at least one colouring agent, in particular at least one pigment,

D) exposure of the coated nail or false nail obtained at the end of step C) to a UV or visible light radiation,
E) optionally, application, to the second coating resulting from step C) and D), of a third composition, via which a third coating consisting of at least one coat of said third composition is deposited, the third composition comprising:

- at least one urethane (meth)acrylate compound corresponding to formula (XIII) below:

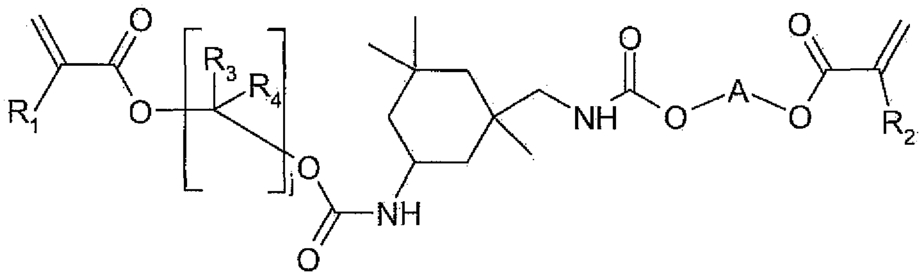

Formula (XIII)

in which formula (XIII):

- $R_1$, $R_2$, $R_3$ and $R_4$, which may be identical or different, represent a hydrogen atom or a $C_1$-$C_{10}$ alkyl chain, preferably a hydrogen atom or a methyl group,
- j is between 1 and 10, preferably equal to 2,
- A represents a $C_1$-$C_{10}$ alkyl group, or a polyurethane comprising from 2 to 20 carbamates units,

- at least one photo-crosslinkable compound b) corresponding to formula (XI) below:

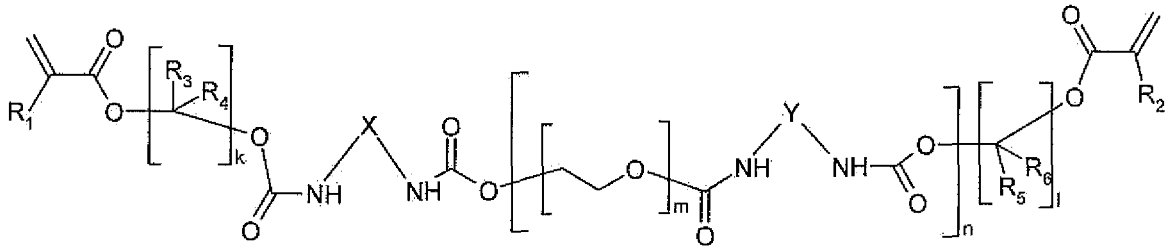

Formula (XI)

in which formula (XI):

- $R_1$, $R_2$, $R_6$, $R_4$, $R_5$, and $R_6$, which may be identical or different, represent a hydrogen atom or a $C_1$-$C_{10}$ alkyl chain, preferably a hydrogen atom or a methyl group,
- with k and 1, which may be identical or different, between 1 and 10, preferably equal to 2,
- with m between 1 and 100, preferably between 5 and 50, preferably between approximately 8 and 10,
- with n between 1 and 10, preferably equal to 1,
- with X and Y, which may be identical or different, representing a $C_1$-$C_{20}$ alkyl or cycloalkyl group,

- at least one photoinitiator, preferably chosen from the group consisting of α-hydroxy ketones, α-amino ketones, aromatic ketones preferably combined with a hydrogen-donating compound, aromatic α-diketones and acylphosphine oxides, and mixtures thereof, advantageously from the group consisting of acylphosphine oxides,

- preferably at least one monofunctional (meth)acrylate monomer, preferably tetrahydrofurfuryl methacrylate (THFMA),

F) exposure of the coated nail or false nail obtained at the end of step E) to a UV or visible light radiation.

The present invention relates more particularly to a method for coating the nails and/or false nails, in which, when a third coating of a third composition is applied, the second composition applied as second coating comprises at least one colouring agent.

The present invention relates more particularly to a method for coating the nails and/or false nails, in which steps C) and D) are repeated in total twice, the second composition being applied a first time and exposed to a UV or visible light radiation, then being applied a second time and exposed to a UV or visible light radiation, steps A) and B), like E) and F), preferably being respectively carried out only once.

In such methods, the second coating is preferably a or preferentially a plurality of coloured coat(s), comprising at least one colouring agent, and the third coating is preferably a top coat free of colouring agent.

***Solids***

A composition according to the invention advantageously comprises a solids content of greater than or equal to 30%, in particular greater than or equal to 40%, and advantageously less than or equal to 60%, in particular less than or equal to 50%.

For the purposes of the present invention, the "*solids content*" denotes the content of non-volatile matter.

The solids content (abbreviated as SC) of a composition according to the invention is measured using a "Halogen Moisture Analyzer HR 73" commercial halogen desiccator from Mettler Toledo. The measurement is performed on the basis of the weight loss of a sample dried by halogen heating, and thus represents the percentage of residual matter once the volatile matter has evaporated off.

The measurement protocol is as follows:

About 2 g of the composition, hereinafter the sample, are spread onto a metal crucible. The sample is photo-crosslinked under a nitrogen stream (in order to prevent the atmospheric oxygen from inhibiting the crosslinking at the surface of the sample). The metal crucible is then placed in the halogen moisture analyzer mentioned above. The sample is then subjected to a temperature of 105°C until a constant weight is obtained. The wet mass of the sample, corresponding to its initial mass before crosslinking, and the dry mass of the sample, corresponding to its mass after crosslinking and halogen heating, are measured using a precision balance.

The experimental error associated with the measurement is of the order of plus or minus 2%.

The solids content is calculated in the following manner:

$$\text{Solids content (expressed as weight percentage)} = 100 \times (\text{dry mass/wet mass}).$$

***Physiologically acceptable medium***

The cosmetic compositions according to the invention comprise a physiologically acceptable medium.

The term "physiologically acceptable medium" is intended to denote a medium that is particularly suitable for applying a composition of the invention to keratin materials.

The physiologically acceptable medium is generally adapted to the nature of the support onto which the composition has to be applied, and also to the appearance under which the composition has to be packaged.

***Photo-crosslinkable compounds***

A composition in accordance with the present invention comprises at least the photo-crosslinkable compounds a) and b).

In the context of the present invention, the term "photo-crosslinkable compounds" denotes organic compounds capable of crosslinking under the action of light radiation, resulting in a crosslinked polymeric network.

***Photo-crosslinkable compound a)***

The compositions according to the invention comprise at least one photo-crosslinkable compound a). They can therefore comprise a single photo-crosslinkable compound or a mixture of several proto-crosslinkable compounds.

According to one embodiment, the compositions of the invention comprise a single photo-crosslinkable compound as defined hereinafter.

In the context of the present invention, the term "photo-crosslinkable compound" denotes an organic compound capable of crosslinking under the action of light radiation, resulting in a crosslinked polymeric network.

According **to the invention,** the photo-crosslinkable compound(s) a) correspond(s).

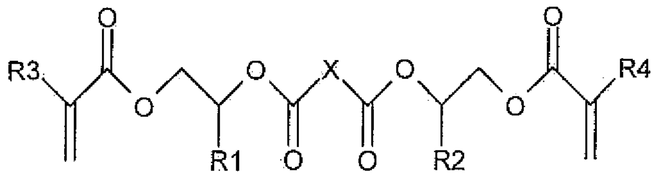

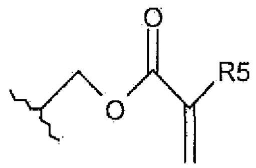

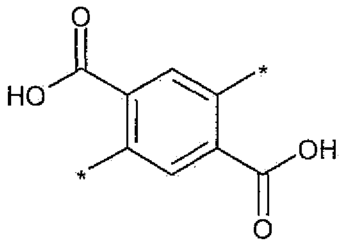

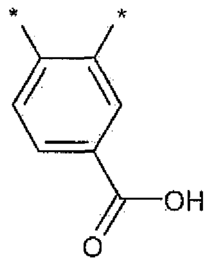

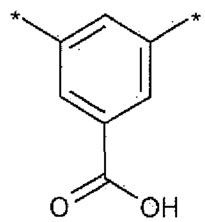

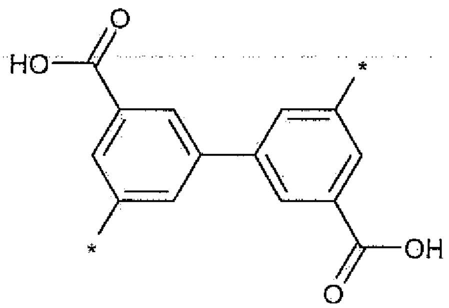

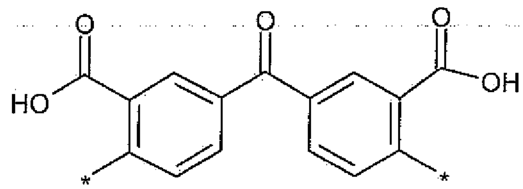

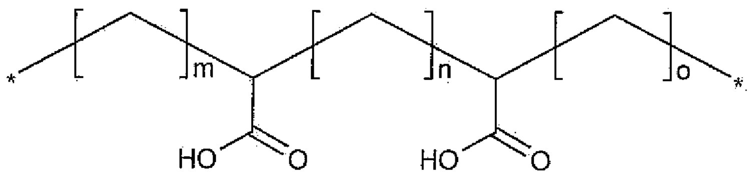

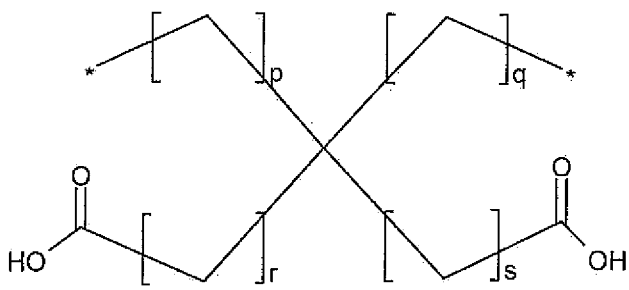

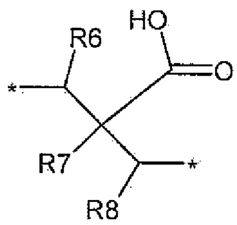

to formula (I-1) below:

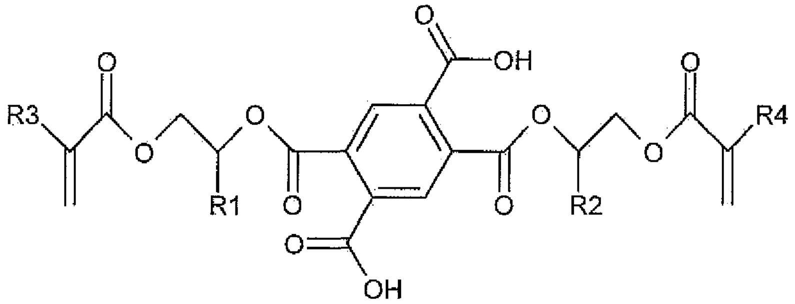

(I-1)

in which :
R3 and R4, which may be identical or different, represent a hydrogen atom or a methyl group and preferably are methyl groups; and
R1 and R2, which may be identical or different, represent a hydrogen atom, a methyl group or a group of formula (II-1) below:

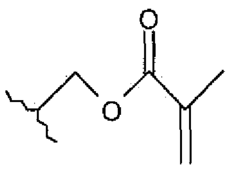

(II-1)

R1 and R2 preferably representing a hydrogen atom.

Preferentially, the photo-crosslinkable compound(s) a) correspond(s) to formula (I-2) below:

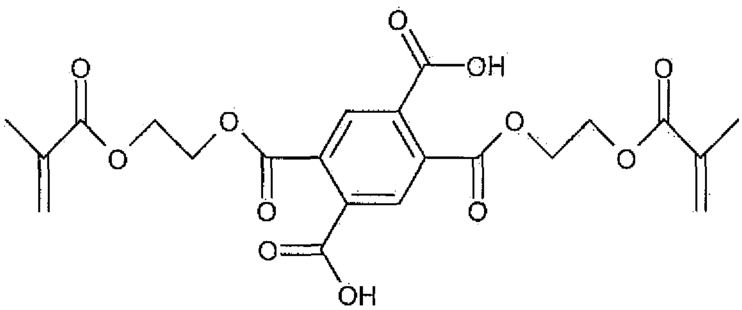

(I-2)

According to one embodiment, the compositions according to the invention have a total content of compound(s) a) greater than or equal to 10% by weight, relative to the total weight of the composition, in particular ranging from 10% to 25% by weight, and more particularly between 15% and 20% by weight, relative to the total weight of the solids of the composition.

***Photo-crosslinkable compound(s) b)***

A composition according to the invention comprises at least one photo-crosslinkable compound b).

The photo-crosslinkable compound(s) b) correspond(s) to formula (XI) below:

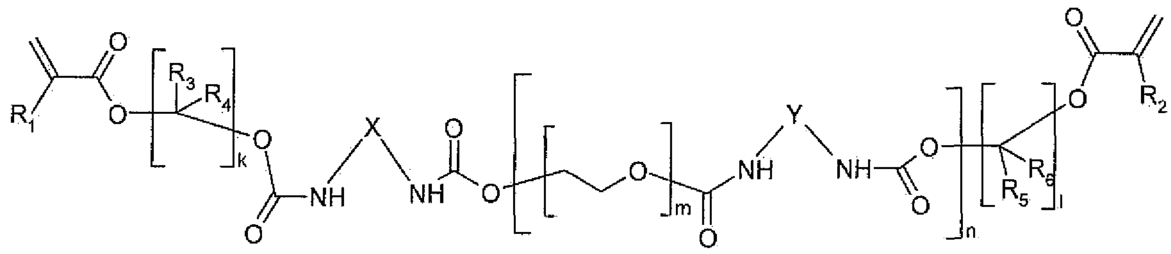

Formula (XI)

in which formula (XI):

- $R_1$, $R_2$, $R_6$, $R_4$, $R_5$, and $R_6$, which may be identical or different, represent a hydrogen atom or a $C_1$-$C_{10}$ alkyl chain, preferably a hydrogen atom or a methyl group,
- k and l, which may be identical or different, are between 1 and 10, preferably equal to 2,
- m is between 1 and 100, preferably between 5 and 50, preferably between approximately 8 and 10,
- n is between 1 and 10, preferably equal to 1,
- X and Y, which may be identical or different, represent a $C_1$-$C_{20}$ alkyl or cycloalkyl group.

This (These) photo-crosslinkable compound(s) b) of formula (XI), advantageously has (have) a molecular weight greater than or equal to 1000 g/mol, in particular ranging from 1000 to 5000 g/mol, preferably from 1000 to 3000 g/mol.
The photo-crosslinkable compound(s) b) of formula (XI), is (are) preferably present in a total content greater than or equal to 20% by weight, relative to the total weight of solids of the composition, in particular between 25% and 50% by weight, and more particularly between 30% and 50% by weight, relative to the total weight of the solids of the composition.
The photo-crosslinkable compound(s) a) of formula (I-1), and the photo-crosslinkable compound(s) b) of formula (XI), is (are) preferably present in a respective total content such that the weight ratio of the photo-crosslinkable compound(s) a) of formula (I-1), and of the photo-crosslinkable compound(s) b) of formula (XI), ranges from 0.1 to 2, in particular from 0.25 to 1.

***Film-forming polymer(s)***

The compositions according to the invention advantageously also comprise at least one film-forming polymer.
They can comprise a single film-forming polymer or a mixture of several film-forming polymers, preferably a mixture of several film-forming polymers.
The function of the film-forming polymer(s) is to confer a wear property on the photo-crosslinkable composition and also to promote removal of the makeup.
Preferably, the compositions according to the invention comprise at least two film-forming polymers.
The total content of film-forming polymer(s) is preferably greater than or equal to 5% by weight, relative to the total weight of solids of the composition, in particular from 10% to 25% by weight, relative to the total weight of solids of the composition.
For the purposes of the present invention, the term "film-forming polymer" denotes a polymer that is capable, by itself (i.e. in the absence of an auxiliary film-forming agent or of an external stimulus for example of the UV type), of forming an isolable and in particular continuous and adherent film, on a support, in particular on the nails.
This film-forming polymer may be chosen from the group consisting of synthetic polymers, of radical type or of polycondensate type, and polymers of natural origin, and mixtures thereof.
A film-forming polymer that is suitable for the invention can be chosen from at least one poly(meth)acrylate compound, in particular from (meth)acrylate homopolymers and copolymers, preferably from (meth)acrylate copolymers.
The poly(meth)acrylate compound(s), in particular the (meth)acrylate copolymer(s), present in the composition is (are) advantageously capable of being obtained by:

i) polymerisation of at least one methyl methacrylate (MMA) monomer and of at least one acrylic or methacrylic acid (AA or MAA) monomer, or
ii) polymerisation of at least one methyl methacrylate (MMA) monomer, of at least one monomer with a glass transition temperature below 30°C, such as butyl methacrylate (BMA), butyl acrylate (BA) or 2-ethylhexyl acrylate (2-EHA), and optionally of at least one acrylic acid (AA) or methacrylic acid (MAA) monomer.

A composition according to the invention preferably comprises at least one poly(meth)acrylate film-forming polymer of type ii) obtained by polymerisation of at least one methyl methacrylate (MMA) monomer, of at least one monomer with a glass transition temperature below 30°C, such as butyl methacrylate (BMA), butyl acrylate (BA) or 2-ethylhexyl acrylate (2-EHA), and optionally of at least one acrylic acid (AA) or methacrylic acid (MAA) monomer.
Such a poly(meth)acrylate film-forming polymer of type ii) preferably corresponds to formula (XII) below:

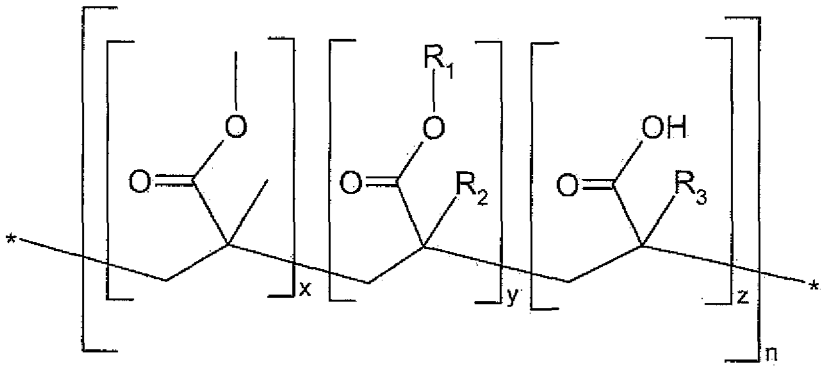

Formula (XII)

in which formula (XII):

- $R_1$, $R_2$ and $R_3$, which may be identical or different, represent a hydrogen atom or a $C_1$-$C_{10}$ alkyl group, $R_1$ preferably representing a $C_4$-$C_{10}$ alkyl group, and $R_2$ and $R_3$ preferably representing a hydrogen atom or a methyl group,
- x and y, which may be identical or different, represent an integer between 1 and 100,
- z represents an integer between 0 and 100,
- n represents an integer between 1 and 1000.

Preferably, a composition according to the invention comprises at least one film-forming polymer c) chosen from at least one polyacrylate compound of formula (XII).
As a variant or preferably additionally, a film-forming polymer that is suitable for the invention can be chosen from polysaccharides and polysaccharide derivatives, such as derivatives of cellulose or of guar gum. A preferential polysaccharide derivative that is suitable for the invention may be nitrocellulose or a polysaccharide ester or alkyl ether.
The term "polysaccharide ester or alkyl ether" denotes a polysaccharide made up of repeat units comprising at least two identical or different rings and having a degree of substitution per saccharide unit of between 1.9 and 3, preferably between 2.2 and 2.9 and more particularly between 2.4 and 2.8. The term "substitution" denotes the functionalisation of the hydroxyl groups to give ester and/or alkyl ether functions, and/or the functionalisation of the carboxylic groups to give ester functions.
In other words, it may be a polysaccharide, partially or totally substituted with ester and/or alkyl ether groups. Preferably, the hydroxyl groups may be substituted with ester and/or alkyl ether functions of $C_2$-$C_4$.
Mention may in particular be made of cellulose esters (such as cellulose acetobutyrates or cellulose acetopropionates), cellulose alkyl ethers (for instance ethylcelluloses), and ethyl guars.
A film-forming polymer that is suitable for the invention can be chosen from synthetic polymers such as polyurethanes, acrylic polymers, vinyl polymers, polyvinyl butyrals, alkyd resins and ketone/aldehyde resins, resins derived from aldehyde condensation products, such as arylsulfonamide-formaldehyde resins, for instance toluenesulfonamide-formaldehyde resin, arylsulfonamide-epoxy resins or else ethyl tosylamide resins.
A film-forming polymer that is suitable for the invention can also be chosen from polymers of natural origin, such as plant resins, such as dammar resins, elemi resins, copal resins, and benzoin; gums such as shellac, sandarac gum and gum mastic.
Use may in particular be made, as a film-forming polymer, of the toluenesulfonamide/formaldehyde resins Ketjentflex MS80 from the company Akzo or Santolite MHP or Santolite MS 80 from the company Faconnier or Resimpol 80 from the company Pan Americana, the alkyd resin Beckosol ODE 230-70-E from the company Dainippon, the acrylic resin Acryloid B66 from the company Röhm & Haas, the polyurethane resin Trixene PR 4127 from the company Baxenden or the acetophenone/formaldehyde resin sold under the reference Synthetic Resin SK by Degussa.
According to one particular preferred embodiment, the film-forming polymer is chosen from the group consisting of polysaccharides and polysaccharide derivatives, preferably from nitrocellulose and polysaccharide ethers and esters, in particular of $C_2$-$C_4$, and more preferentially from cellulose acetobutyrates, cellulose acetopropionates, ethylcelluloses,

ethyl guars and mixtures thereof.

According to one advantageous embodiment, the film-forming polymer is chosen from the group consisting of nitrocellulose, cellulose acetopropionate, cellulose acetobutyrate, and (meth)acrylate homopolymers and copolymers, and mixtures thereof.

According to one advantageous embodiment, the compositions of the invention comprise at least one film-forming polymer chosen from nitrocellulose.

The film-forming polymer(s), in particular chosen from the group consisting of poly(meth)acrylates, in particular corresponding to formula (XII), is (are) present in a total content greater than or equal to 10% by weight, relative to the total weight of solids of the composition, in particular a content ranging from 13% to 20% by weight, relative to the total weight of solids of the composition.

The film-forming polymer(s), in particular chosen from the group consisting of polysaccharides or polysaccharide derivatives is (are) present in a total content greater than or equal to 5% by weight, relative to the total weight of solids of the composition, in particular a content ranging from 10% to 15% by weight, relative to the total solids of the composition.

According to this particular embodiment, the ratio of the weight of the film-forming polymer(s) chosen from the group consisting of polysaccharides and polysaccharide derivatives, in particular the weight of nitrocellulose, to the weight of the photo-crosslinkable compounds, in particular the sum of the respective weight of the photo-crosslinkable compounds a) and b), is less than or equal to 1, and preferentially between 0.5 and 1.

According to one advantageous embodiment, the ratio of the total weight of film-forming polymer(s), in particular chosen from the group consisting of poly(meth)acrylate compound(s), in particular of formula (XII), and the polysaccharides and polysaccharide derivatives, in particular nitrocellulose, to the weight of the photo-crosslinkable compounds, in particular the sum of the respective weight of the photo-crosslinkable compounds a) and b), and optionally of one or more (meth)acrylate monomer(s), such as a tetrahydrofurfuryl methacrylate compound, is less than or equal to 1, and preferentially between 0.5 and 1.

***Volatile solvent(s)***

The compositions according to the invention also advantageously comprise at least one volatile solvent. They can therefore comprise a single solvent or a mixture of several volatile solvents, preferably a mixture of several volatile solvents.

The weight content of volatile solvents is preferably between 40% and 80% and preferably between 50% and 70% by weight, relative to the total weight of the composition.

For the purposes of the invention, the term "volatile solvent" is intended to mean a solvent that is capable of evaporating on contact with keratin materials in less than one hour, at ambient temperature and atmospheric pressure.

The volatile solveht(s) of the invention are solvents which are liquid at ambient temperature and which have a non-zero vapour pressure, at ambient temperature and atmospheric pressure, ranging in particular from 50 Pa to 40 000 Pa (0.375 to 300 mmHg), in particular ranging from 100 Pa to 26 6624 Pa (0.75 to 200 mmHg) and more particularly ranging from 1000 Pa to 13 332 Pa (7.5 to 100 mmHg).

Such solvents aim in particular to fluidize and reduce the solids of the composition.

Preferably, the solvents are chosen from polar solvents.

For the purposes of the present invention, the term "polar solvent" is intended to mean a solvent, or an oil, of which the solubility parameter calculated above its melting point $\delta_a$ is other than 0 $(J/cm^3)^{1/2}$.

The definition and calculation of the solubility parameters in the Hansen three-dimensional solubility space are described in the article by C.M. Hansen: "The three dimensional solubility parameters" J. Paint Technol. 39, 105 (1967).

According to this Hansen space:

- $\delta_D$ characterizes the London dispersion forces derived from the formation of dipoles induced during molecular impacts;
- $\delta_p$ characterizes the Debye interaction forces between permanent dipoles and also the Keesom interaction forces between induced dipoles and permanent dipoles;
- $\delta_h$ characterizes the specific interaction forces (such as hydrogen bonding, acid/base, donor/acceptor, etc.); and
- $\delta_a$ is determined by the equation: $\delta_a = (\delta_p^2 + \delta_h^2)^{1/2}$.

The parameters $\delta_p$, $\delta_h$, $\delta_D$ and $\delta_a$ are expressed in $(J/cm^3)^{1/2}$.

In particular, the term "polar solvent" is intended to mean a solvent of which the chemical structure is formed essentially from, or even consists of, carbon and hydrogen atoms, and which comprises at least one highly electronegative heteroatom such as an oxygen, nitrogen, silicon or phosphorus atom.

Preferably, this polar volatile solvent is chosen from the group consisting of $C_3$-$C_6$ esters and ketones and mixtures thereof

By way of polar volatile solvent, mention may in particular be made of acetone, methyl ethyl ketone, methyl isobutyl ketone, cyclohexanone and alkyl acetates in which the alkyl group comprises from 2 to 5 carbon atoms, such as methyl acetate, ethyl acetate, propyl acetate, n-propyl acetate, isopropyl acetate, n-butyl acetate, isobutyl acetate and tert-butyl acetate.

Preferably, the polar volatile solvent is chosen from the group consisting of ethyl acetate, propyl acetate, such as n-propyl or isopropyl acetate, n-butyl, isobutyl or tert-butyl acetate, isopropanol, and a mixture or mixtures thereof.

According to one preferred embodiment, the solvent is a mixture of butyl acetate, ethyl acetate and isopropanol.

The butyl acetate, the ethyl acetate and the isopropanol are preferably present in a respective content ranging respectively from 25% to 35% by weight, from 20% to 30% by weight, and from 1% to 5%, relative to the total weight of the composition.

According to another preferred embodiment, the solvent is a mixture of butyl acetate, ethyl acetate, propyl acetate and isopropanol.

The butyl acetate, the ethyl acetate, the propyl acetate and the isopropanol are preferably present in a respective content ranging respectively from 20% to 30% by weight, from 15% to 25% by weight, from 5% to 15% by weight, and from 1% to 5%, relative to the total weight of the composition.

***Photoinitiator(s)***

The compositions according to the invention also advantageously comprise at least one photoinitiator.

It can comprise a single photoinitiator or a mixture of several photoinitiators, preferably a single photoinitiator.

The photoinitiators that can be used according to the present invention are known in the art and are described, for example, in "Les photoinitiateurs dans la réticulation des revêtements" ["Photoinitiators in the crosslinking of coatings"], G. Li Bassi, Double Liaison - Chimie des Peintures, n°361, November 1985, p.34-41; "Applications industrielles de la polymerisation photoinduite" ["Industrial applications of photoinduced polymerisation"], Henri Strub, L'Actualité Chimique, February 2000, p.5-13; and "Photopolymères: considerations théoriques et réaction de prise" ["Photopolymers: theoretical considerations and setting reaction"], Marc, J.M. Abadie, Double Liaison - Chimie des Peintures, n°435-436, 1992, p.28-34.

These photoinitiators encompass:

- α-hydroxy ketones, sold, for example, under the names Darocur® 1173 and 4265, Irgacure® 184, 2959, and 500 by the company BASF, and Additol® CPK by the company Cytec,
- α-amino ketones, sold, for example, under the names Irgacure® 907 et 369 by the company BASF,
- aromatic ketones, sold, for example, under the name Esacure® TZT by Lamberti. Mention may also be made of the thioxanthones sold, for example, under the name Esacure® ITX by Lamberti, and quinones. These aromatic ketones usually require the presence of a hydrogen-donating compound such as tertiary amines and in particular alkanolamines. Mention may in particular be made of the tertiary amine Esacure® EDB sold by the company Lamberti;
- α-dicarbonyl derivatives, the most common representative of which is benzyl dimethyl ketal, sold under the name Irgacure® 651 by BASF. Other commercial products are sold by the company Lamberti under the name Esacure® KB1, and
- acylphosphine oxides such as, for example, the bis-acylphosphine oxides (BAPOs) sold, for example, under the names Irgacure® 819, 1700 and 1800, Darocur® 4265, Lucirin® TPO and Lucirin® TPO-L by the company BASF.

Preferably, the photoinitiator is chosen from the group consisting of α-hydroxy ketones, α-amino ketones, aromatic ketones preferably combined with a hydrogen-donating compound, aromatic α-diketones and acylphosphine oxides, and mixtures thereof.

An acylphosphine oxide is preferably used in the photo-crosslinkable composition of the invention.

By way of photoinitiator, mention may be made of Lucirin® TPO-L (BASF).

The total content of the photoinitiator(s) depends on a large number of factors such as, for example, the reactivity of the various components of the mixture, the presence of a colouring agent or colouring agents, the intensity of the light source or the exposure time.

In order to obtain the desired properties, the photoinitiator(s) is (are) preferably present in a total content greater than or equal to 0.1% by weight, relative to the total weight of the photo-crosslinkable composition, preferably ranging from 0.2% to 5% by weight, relative to the total weight of the photo-crosslinkable composition.

***Other constituents***

According to one particular embodiment, a composition according to the invention may also comprise one or more (meth)acrylate monomer(s), such as a tetrahydrofurfuryl methacrylate compound. In particular, this (these)

(meth)acrylate monomer(s) may be present in a second coat applied to the base coat in order to improve the wear property and the mechanical property. Such a monomer can, however, be present in a base coat in accordance with the invention. This (these) monomer(s) is (are) preferably present in a content less than or equal to 10% by weight, relative to the total weight of the solids of the composition, better still in a content less than or equal to 5% by weight, relative to the total weight of the solids of the composition.

According to one particular embodiment, a composition according to the invention may also comprise at least one photo-crosslinkable (poly)urethane (meth)acrylate compound not comprising a polyoxyalkylene unit.

The compositions according to the invention may also comprise one or more stabiliser(s).

The compositions according to the invention can also contain adjuvants, or additives, chosen in particular from colouring agents such as pigments, plasticisers, coalescers, preservatives, thickeners, fragrances, cosmetic nail care active agents, spreading agents, antifoams and dispersants.

Needless to say, those skilled in the art will take care to choose these optional adjuvants and additives such that the advantageous properties of the composition according to the invention are not, or are virtually not, adversely affected by the envisaged addition.

When the composition comprises colouring agents, the absorption spectrum of the colouring agents used should in particular be adapted to that of the photoinitiators, or conversely the absorption spectrum of the photoinitiators to that of the colouring agents used, in order to avoid these two types of compounds absorbing light at the same wavelengths. This is because the absorption of light by the colouring agents would render almost totally ineffective the photoinitiators present beyond a certain depth of the coating.

Preferably, the composition of the invention is transparent.

As used herein, the term "transparent" means that the composition has HAZEBYK index of less than 5 as measured with a KYKHAZEGLOSS brilliance meter.

According to one embodiment, the composition of the invention also comprises a colouring agent chosen from the group consisting of soluble dyes, pigments, nacres and glitter flakes.

The colouring agent(s) can be present in a total content greater than or equal to 0.1% by weight, relative to the total weight of the coat, preferably ranging from 0.1% to 5%, advantageously from 0.2% to 1% by weight, relative to the total weight of the composition.

The term "soluble dyes" should be understood as meaning organic, inorganic or organometallic compounds which are soluble in the composition of the invention and intended to colour said composition.

The dyes are, for example, Sudan red, DC red 17, DC green 6, β-carotene, soybean oil, Sudan brown, DC yellow 11, DC violet 2, DC orange 5 and quinoline yellow.

The term "pigments" should be understood as meaning white or coloured and inorganic or organic particles of any shape which are insoluble in the composition of the invention and which are intended to colour said composition.

The term "nacres" should be understood as meaning iridescent particles of any shape, in particular produced by certain molluscs in their shell, or else synthesized.

The pigments may be white or coloured, and inorganic and/or organic. Among the inorganic pigments that may be mentioned are titanium dioxide, optionally surface-treated, zirconium oxide or cerium oxide, and also zinc oxide, iron (black, yellow or red) oxide or chromium oxide, manganese violet, ultramarine blue, chromium hydrate and ferric blue, and metal powders, for instance aluminium powder and copper powder.

Among the organic pigments that may be mentioned are carbon black, pigments of D & C type and lakes based on cochineal carmine or on barium, strontium, calcium or aluminium.

Mention may also be made of effect pigments, such as particles comprising an organic or inorganic and natural or synthetic substrate, for example glass, acrylic, polyester, polyurethane or polyethylene terephthalate resins, ceramics or aluminas, which may or may not be covered with metal substances, such as aluminium, gold, copper or bronze, or with metal oxides, such as titanium dioxide, iron oxide or chromium oxide, or with inorganic or organic pigments, and mixtures thereof.

The nacreous pigments can be chosen from white nacreous pigments, such as mica covered with titanium oxide or with bismuth oxychloride, coloured nacreous pigments, such as titanium oxide-coated mica covered with iron oxides, titanium oxide-coated mica covered with in particular ferric blue or with chromium oxide, or titanium oxide-coated mica covered with an organic pigment of the abovementioned type, and nacreous pigments based on bismuth oxychloride.

Use may also be made of pigments with goniochromatic properties, in particular with liquid crystals or multilayer pigments.

Optical brighteners or fibres optionally coated with optical brighteners can also be used.

The compositions according to the invention may also comprise one or more fillers, in particular in a content ranging from 0.01%to 50% by weight, relative to the total weight of the composition, and preferably ranging from 0.01% to 30% by weight.

The term "fillers" should be understood as meaning colourless or white, inorganic or synthetic particles of any shape, which are insoluble in the medium of the composition, irrespective of the temperature at which the composition

is manufactured. These fillers serve in particular to modify the rheology or the texture of the composition.

The fillers may be inorganic or organic and of any shape, platelet-shaped, spherical or oblong, irrespective of the crystallographic form (for example lamellar, cubic, hexagonal, orthorhombic, etc.). Mention may be made of talc, mica, silica, kaolin, polyamide (Nylon®) powder (Orgasol® from Atochem), poly-β-alanine powder and polyethylene powder, powders of tetrafluoroethylene polymers (Teflon®), lauroyllysine, starch, boron nitride, hollow polymer microspheres such as those of polyvinylidene chloride/acrylonitrile, for instance Expancel® (Nobel Industrie) or of acrylic acid copolymers (Polytrap® from the company Dow Corning) and silicone resin microbeads (for example Tospearls® from Toshiba), elastomeric polyorganosiloxane particles, precipitated calcium carbonate, magnesium carbonate, magnesium hydrogen carbonate, hydroxyapatite, hollow silica microspheres (Silica Beads® from Maprecos), glass or ceramic microcapsules, and metal soaps derived from organic carboxylic acids containing from 8 to 22 carbon atoms and preferably from 12 to 18 carbon atoms, for example zinc stearate, magnesium stearate or lithium stearate, zinc laurate or magnesium myristate.

***Uses***

According to one embodiment, the compositions of the invention are intended to be applied to the nails and/or false nails, preferably for making up and/or caring for the nails and/or false nails, more preferentially for making up the nails and/or false nails.

In particular, the compositions according to the invention are intended to be used as photo-crosslinkable nail varnish.

Preferably, the compositions according to the invention are intended to be applied directly to the nails and/or false nails as a base coat or coating. Such a base coating can optionally constitute a first coating for one or more subsequent coating(s).

The present invention also relates to a method for making up and/or caring for the nails and/or false nails, consisting in applying, to a nail and/or a false nail, a photo-crosslinkable composition according to the invention.

According to one particular embodiment, a method for coating the nails and/or false nails, in particular for making up and/or caring for the nails and/or false nails, comprises at least the following steps:

A) application, to a nail or a false nail, of a composition in accordance with the invention, via which a coating consisting of at least one coat of said photo-crosslinkable composition is deposited, this coating being applied directly in contact with the nail or the false nail, and

B) exposure of the coated nail or false nail obtained at the end of step A) to a UV or visible light radiation.

The radiation suitable for crosslinking the photo-crosslinkable composition of the present invention has a wavelength between 210 and 600 nm, preferably between 250 and 420 nm, preferably between 350 and 410 nm. The use of lasers may also be envisaged.

In one preferred embodiment of the invention, use is made of an LED lamp or a UV lamp and in particular a mercury-vapour lamp, the mercury optionally being doped with other elements, such as gallium, making it possible to modify the emission spectrum of the light source.

The radiation-exposure time of the deposited coating depends on various factors such as the chemical nature and the content of the reactive components or else the desired crosslinking density.

For nail varnishes, it will generally be sought to obtain satisfactory results for an exposure time between 10 seconds and 10 minutes, preferably between 30 seconds and 5 minutes.

Such a method can use a UV lamp with a power of approximately 36 W.

Preferably, the thickness after drying of the coating of photo-crosslinkable composition deposited in step A) is less than or equal to 100 μm and preferably less than or equal to 75 μm.

At the end of the final crosslinking step, the coating deposited on the nail or the false nail can exhibit a tacky coat at its surface, requiring cleaning of the crosslinked coating using, for example, a solvent such as isopropanol.

According to one embodiment, the method of the invention also comprises, before step B), a period of drying the coating deposited at the end of step A), the duration of which can range from 10 seconds to 10 minutes, typically from 30 seconds to 5 minutes. Said drying is generally carried out in the open air and at ambient temperature.

A particular method according to the invention consists exclusively of a step A) and a step B) as defined above, optionally separated by a drying period as defined above.

According to one embodiment, after the crosslinking step B), the coating deposited on the nail is covered with at least one coloured composition and/or with a top composition, also known as a "top coat", these compositions being photo-crosslinkable or non-photo-crosslinkable.

The crosslinked coating resulting from the crosslinking of step B) exhibits a persistence over time, in terms of resistance to chipping and of gloss, which is significant and in particular on the scale of at least one week. It thus proves

to be resistant to water, to rubbing and to impacts, and does not exhibit any significant wear or chipping during this period.

This coating also has an ability to dissolve or to increase in volume and therefore in weight when it is brought into contact with a customary makeup-removing solvent. This ability to dissolve or to swell, shown by the crosslinked coating, is precisely advantageous for its removal when it is applied at the surface of a nail or of a false nail. Indeed, the coating can be easily removed by simple makeup removal using a conventional dissolving agent.

Thus, the composition of the invention is advantageously removable using dissolving agents which are customary in the nail varnish field, and in particular using acetone and ethyl acetate, and mixtures thereof.

The present invention also relates to a method for removing the makeup from the nails and/or false nails, comprising the application of a makeup-removal composition, such as a customary dissolving agent described above, to a nail or a false nail coated with at least one coat obtained by crosslinking a coat of composition according to the invention, via which said crosslinked coat is removed.

The first coating, or base coating, is preferably coated with a second coating. In particular, this second coating is chosen from a top coating or a coloured coating. More particularly, the first coating can be coated with a coloured coating as second coating, and the second coating can itself be coated with a top coating as third coating. Preferably, each coating consists of a respective photo-crosslinkable composition and is the subject of photo-crosslinking according to the conditions set out above.

According to one particular embodiment, a method for coating the nails and/or false nails, in particular for making up and/or caring for the nails and/or false nails, comprises at least the following steps:

A) application, to a nail or a false nail, of a first composition in accordance with the invention, via which a first coating consisting of at least one coat of said photo-crosslinkable composition is deposited, this first coating being applied directly in contact with the nail or the false nail, and
B) exposure of the coated nail or false nail obtained at the end of step A) to a UV or visible light radiation,
C) application, to the first coating resulting from step A) and B), of a second composition, distinct from the first composition, via which a second coating consisting of at least one coat of said second composition is deposited,
D) exposure of the coated nail or false nail obtained at the end of step C) to a UV or visible light radiation.

In such a method, the second coating is preferably a top coat, optionally free of colouring agent.

According to one particular embodiment, a method for coating the nails and/or false nails, in particular for making up and/or caring for the nails and/or false nails, comprises at least the following steps:

A) application, to a nail or a false nail, of a first composition in accordance with the invention, via which a first coating consisting of at least one coat of said photo-crosslinkable composition is deposited, this first coating being applied directly in contact with the nail or the false nail, and
B) exposure of the coated nail or false nail obtained at the end of step A) to a UV or visible light radiation,
C) application, to the first coating resulting from step A) and B), of a second composition, distinct from the first composition, via which a second coating consisting of at least one coat of said second composition is deposited,
D) exposure of the coated nail or false nail obtained at the end of step C) to a UV or visible light radiation.
E) application, to the second coating resulting from step C) and D), of a third composition, distinct from the first composition and from the second composition, via which a third coating consisting of at least one coat of said third composition is deposited,
F) exposure of the coated nail or false nail obtained at the end of step E) to a UV or visible light radiation.

In such a method, the second coating is preferably a coloured coat comprising at least one colouring agent and the third coating is preferably a top coat free of colouring agent.

According to one preferred embodiment, the present invention thus relates to a method for coating the nails and/or false nails, in particular for making up and/or caring for the nails and/or false nails, comprising at least the following steps:

A) application, to a nail or a false nail, of a first composition in accordance with the invention, via which a first coating consisting of at least one coat of said photo-crosslinkable composition is deposited,
B) exposure of the coated nail or false nail obtained at the end of step A) to a UV or visible light radiation,
C) application, to the first coating resulting from step A) and B), of a second composition, distinct from the first composition, via which a second coating consisting of at least one coat of said second composition is deposited, the second composition comprising:

- at least one urethane (meth)acrylate compound corresponding to formula (XIII) below:

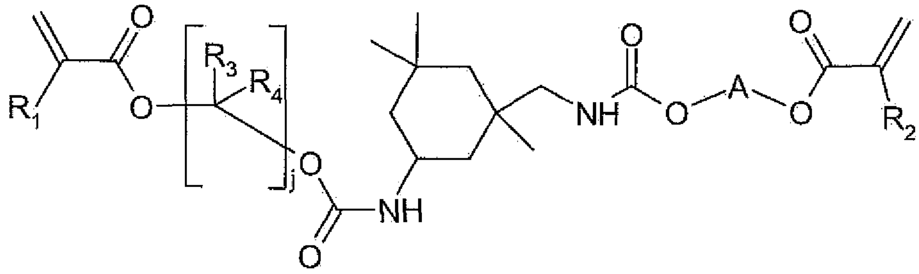

Formula (XIII)

in which formula (XIII):

- $R_1$, $R_2$, $R_3$ and $R_4$, which may be identical or different, represent a hydrogen atom or a $C_1$-$C_{10}$ alkyl chain, preferably a hydrogen atom or a methyl group,
- with j between 1 and 10, preferably equal to 2,
- with A representing a $C_1$-$C_{10}$ alkyl group, or a polyurethane comprising from 2 to 20 carbamate units,

- at least one photo-crosslinkable compound b) comprising at least one urethane poly(ALK)acrylate compound, preferably urethane poly(meth)acrylate compound, comprising a (poly)oxyalkylene unit, in particular comprising at least one (poly)oxyethylene unit, more particularly comprising from 1 to 100 oxyalkylene units, preferably from 5 to 50 oxyalkylene units, and preferentially approximately 8 to 10 oxyalkylene units, the compound(s) b) corresponding to formula (XI) below:

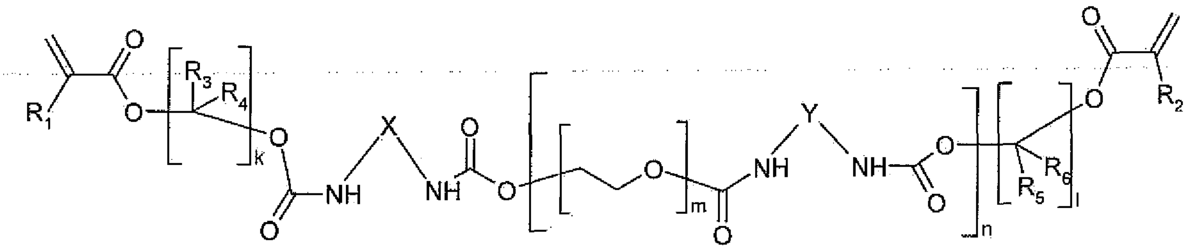

Formula (XI)

in which formula (XI):

- $R_1$, $R_2$, $R_3$, $R_4$, $R_5$, and $R_6$, which may be identical or different, represent a hydrogen atom or a $C_1$-$C_{10}$ alkyl chain, preferably a hydrogen atom or a methyl group,
- with k and l, which may be identical or different, between 1 and 10, preferably equal to 2,
- with m between 1 and 100, preferably between 5 and 50, preferably between approximately 8 and 10,
- with n between 1 and 10, preferably equal to 1,
- with X and Y, which may be identical or different, representing a $C_1$-$C_{20}$ alkyl

or cycloalkyl group,

- at least one photoinitiator, preferably chosen from the group consisting of α-hydroxy ketones, α-amino ketones, aromatic ketones preferably combined with a hydrogen-donating compound, aromatic α-diketones and acylphosphine oxides, and mixtures thereof, advantageously from the group consisting of acylphosphine oxides,
- preferably at least one monofunctional (meth)acrylate monomer, preferably tetrahydrofurfuryl methacrylate (THFMA),
- optionally at least one colouring agent, in particular at least one pigment,

D) exposure of the coated nail or false nail obtained at the end of step C) to a UV or visible light radiation,
E) optionally, application, to the second coating resulting from step C) and D), of a third composition, via which a third coating consisting of at least one coat of said third composition is deposited, the third composition comprising:

- at least one urethane (meth)acrylate compound comprising at least 2 carbamate units obtained by reaction with

at least one diisocyanate of isophorone diisocyanate (IPDI) type, preferably corresponding to formula (XIII) below:

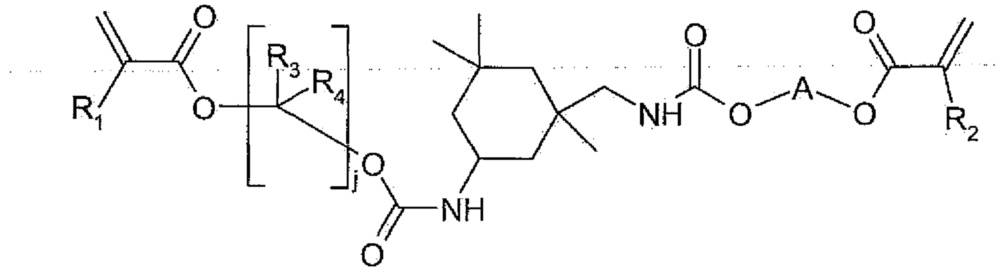

Formula (XIII)

in which formula (XIII):

- $R_1$, $R_2$, $R_3$ $R_4$, which may be identical or different, represent a hydrogen atom or a $C_1$-$C_{10}$ alkyl chain, preferably a hydrogen atom or a methyl group,
- with j between 1 and 10, preferably equal to 2,
- with A representing a $C_1$-$C_{10}$ alkyl group, or a polyurethane comprising from 2 to 20 carbamate units,

- at least one photo-crosslinkable compound b) comprising at least one urethane poly(ALK)acrylate compound, preferably urethane poly(meth)acrylate compound, comprising a (poly)oxyalkylene unit, in particular comprising at least one (poly)oxyethylene unit, more particularly comprising from 1 to 100 oxyalkylene units, preferably from 5 to 50 oxyalkylene units, and preferentially approximately 8 to 10 oxyalkylene units, the compound(s) b) corresponding to formula (XI) below:

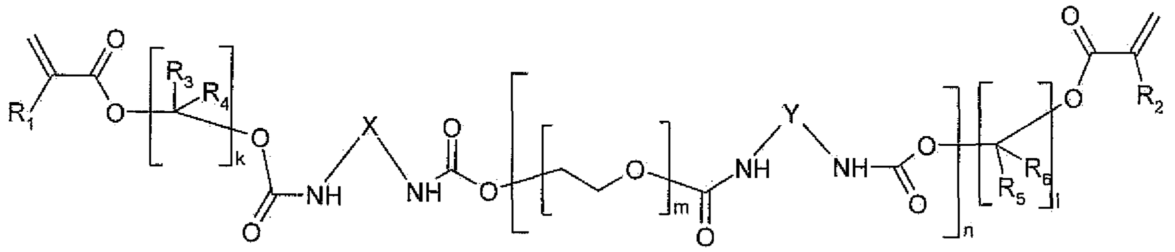

Formula (XI)

in which formula (XI):

- $R_1$, $R_2$, $R_3$, $R_4$, $R_5$, and $R_6$, which may be identical or different, represent a hydrogen atom or a $C_1$-$C_{10}$ alkyl chain, preferably a hydrogen atom or a methyl group,
- with k and l, which may be identical or different, between 1 and 10, preferably equal to 2,
- with m between 1 and 100, preferably between 5 and 50, preferably between approximately 8 and 10,
- with n between 1 and 10, preferably equal to 1,
- with X and Y, which may be identical or different, representing a $C_1$-$C_{20}$ alkyl or cycloalkyl group,

- at least one photoinitiator, preferably chosen from the group consisting of α-hydroxy ketones, α-amino ketones, aromatic ketones preferably combined with a hydrogen-donating compound, aromatic α-diketones and acylphosphine oxides, and mixtures thereof, advantageously from the group consisting of acylphosphine oxides,
- preferably at least one monofunctional (meth)acrylate monomer, preferably tetrahydrofurfuryl methacrylate (THFMA),

F) exposure of the coated nail or false nail obtained at the end of step E) to a UV or visible light radiation.

According to one preferred embodiment, when a third coating of a third composition is applied, the second composition applied as second coating comprises at least one colouring agent. According to one particularly preferred embodiment, the second coating corresponds to one or preferably more coloured coats, which are preferably identical,

such as two, comprising at least one colouring agent, and the third coating is preferably a top coat free of colouring agent.

According to one preferred embodiment, steps C) and D) are repeated in total twice, the second composition being applied a first time and exposed to a UV or visible light radiation, then being applied a second time and exposed to a UV or visible light radiation, steps A) and B), like E) and F), preferably being respectively carried out only once.

A subject of the present invention is also a kit comprising:

- a photo-crosslinkable cosmetic composition according to the invention,
- an abrasive material having a particle size of greater than or equal to 200, preferably less than 300, advantageously between 220 and 280, and
- an LED lamp or a UV lamp.

A subject of the present invention is also a method for coating a nail and/or false nail, comprising the following steps:

i) rubbing the surface of a nail or of the false nail with an abrasive material having a particle size of greater than or equal to 200, preferably less than 300, advantageously between 220 and 280,
ii) applying a photo-crosslinkable cosmetic composition according to the invention to the surface of the nail or of false nails which has been rubbed following step i), wherein a coat consisting of at least one coat of said composition according to the invention is deposited,
iii) exposing the coated nail or false nails obtained following step ii) to an LED lamp or a UV lamp, such that the photo-crosslinking is carried out so as to obtain a crosslinked coat.

The rubbing step is carried out for less than 10 seconds, preferably less than 5 seconds, for example for approximately 3 seconds.

The weight percentages given in this application can be categorised as the percentage by weight of dry matter of the compounds used, unless otherwise expressly mentioned.

The invention will be better understood on reading the following description, given solely by way of example.

## EXAMPLE

Composition according to the invention:
According to a first exemplary embodiment, the following first composition was prepared:

| Ingredients of the base coating composition | % Content |
|---|---|
| PYROMELLITIC DIMETHACRYLATE (PMDM X-865-0000 - ESSTECH, Inc.) | 7.5 |
| PEG-400 Urethane dimethacrylate (X-726-0000 - ESSTECH, Inc.) | 16 |
| TETRAHYDROFURFURYL METHACRYLATE (X-958-7446 - ESSTECH, Inc.) | 1.5 |
| METHYL METHACRYLATE (MMA) / BUTYL METHACRYLATE (BMA) COPOLYMER (PARALOID B 66 100% from DOW CHEMICAL) | 7 |
| Nitrocellulose containing 30% of isopropyl alcohol (viscosity: E22 - 1/2s) (IDYL E35 TX IPA 30% from BERGERAC - SNPE) | 7 |
| Ethyl acetate | 21.65 |
| Propyl acetate | 10 |
| Butyl acetate | 25 |
| Photoinitiator ethyl (2,4,6-trimethylbenzoyl) phenylphosphinate (Lucirin TPO-L - BASF) | 4 |
| BHT (DI-(TERT-BUTYL)-4-HYDROXYTOLUENE - NIPANOX BHT from CLARIANT) | 0.35 |

The ingredients of the composition are introduced into an opaque flask and stirred away from light with a laboratory mixer of the Rayneri brand until a homogeneous mixture is obtained. A sheet of aluminium will have been placed over the container beforehand in order to prevent evaporation of the solvents.

On a nail roughened beforehand for less than 5 seconds using a file with a particle size of 280, the first composition described above was applied to said nail so as to form a base coating or base coat.

After application, the nail is placed under a 36 W UV lamp for 3 minutes in order to crosslink the composition so as to form a film.

The following top composition is then prepared:

| Ingredients of the top composition | % Content |
|---|---|
| TETRAHYDROFURFURYL METHACRYLATE (X-958-7446 - ESSTECH, Inc.) | 20 |
| Isophorone Urethane Dimethacrylate (X-851-1066 - ESSTECH, Inc.) | 15 |
| PEG-400 Urethane dimethacrylate (X-726-0000 - ESSTECH, Inc.) | 60 |
| METHYL METHACRYLATE (MMA) / BUTYL METHACRYLATE (BMA) COPOLYMER (PARALOID B 66 100% from DOW CHEMICAL) | 1 |
| Photoinitiator ethyl (2,4,6-trimethylbenzoyl) phenylphosphinate (Lucirin TPO-L - BASF) | 4 |

The ingredients of the composition are introduced into an opaque flask and stirred away from light with a laboratory mixer of the Rayneri brand until a homogeneous mixture is obtained. A sheet of aluminium will have been placed over the container beforehand in order to prevent evaporation of the solvents.

This composition is applied to the first coating (base coating) in the form of one or more coats so as to form a top coating.

After application of each coat, in the case just one, the nail is placed under a 36 W UV lamp for 3 minutes in order to crosslink the composition so as to form a film, this operation being repeated for each coat applied.

After having crosslinked the final coat, the surface is cleaned with cotton wool soaked in isopropanol in order to remove the tacky coat.

According to another exemplary embodiment, prior to this top coating, a coloured composition similar to this top composition, except that this composition additionally comprises one or more colouring agent(s), is applied. Such a coloured composition has the following composition:

| Ingredients of the coloured composition | % Content |
|---|---|
| TETRAHYDROFURFURYL METHACRYLATE (X-958-7446 - ESSTECH, Inc.) | 20 |
| Isophorone Urethane Dimethacrylate (X-851-1066 - ESSTECH, Inc.) | 15 |
| PEG-400 Urethane dimethacrylate (X-726-0000 - ESSTECH, Inc.) | 59 |
| METHYL METHACRYLATE (MMA) / BUTYL METHACRYLATE (BMA) COPOLYMER (PARALOID B 66 100% from DOW CHEMICAL) | 1 |
| Photoinitiator ethyl (2,4,6Arimethylbenzoyl) phenylphosphinate (Lucirin TPO-L - BASF) | 4 |
| Pigment | 1 |

The ingredients of the composition are introduced into an opaque flask and stirred away from light with a laboratory mixer of the Rayneri brand until a homogeneous mixture is obtained. A sheet of aluminium will have been placed over the container beforehand in order to prevent evaporation of the solvents.

After application of the base coating, one or more coats of the coloured composition, in the case two coats, was (were) applied to the base coating. After the application of each coat, the nail is placed under a 36 W UV lamp for 3 minutes in order to crosslink the composition so as to form a coloured coating in the form of a film.

After application of the coloured coating and photo-crosslinking of this coating in the form of a film, the top composition previously described is then applied to this coloured coating in the form of one or more coats, in the case a single coat. After the application of this coat, the nail is placed under a 36 W UV lamp for 3 minutes in order to crosslink the composition so as to form a top coating in the form of a film.

After having crosslinked the final coat, the surface is cleaned with cotton wool soaked in isopropanol in order to remove the tacky coat.

Comparative composition:

| Ingredients of the base coating composition | % Content |
|---|---|
| PYROMELLITIC DIMETHACRYLATE (PMDM X-865-0000 - ESSTECH, Inc.) | 7.5 |
| Di-HEMA trimethylhexyl dicarbamate (X-850-0000 - ESSTECH, Inc.) | 16 |
| TETRAHYDROFURFURYL METHACRYLATE (X-958-7446 - ESSTECH, Inc.) | 1.5 |
| METHYL METHACRYLATE (MMA) / BUTYL METHACRYLATE (BMA) COPOLYMER (PARALOID B 66 100% from DOW CHEMICAL) | 7 |
| Nitrocellulose containing 30% of isopropyl alcohol (viscosity: E22 - 1/2s) (IDYL E35 TX IPA 30% from BERGERAC - SNPE) | 7 |
| Ethyl acetate | 21.65 |
| Propyl acetate | 10 |
| Butyl acetate | 25 |
| Photoinitiator ethyl (2,4,6-trimethylbenzoyl) phenylphosphinate (Lucirin TPO-L - BASF) | 4 |
| BHT (DI-(TERT-BUTYL)-4-HYDROXYTOLUENE - NIPANOX BHT from CLARIANT) | 0.35 |

On a nail roughened beforehand for less than 5 seconds using a file with a particle size of 280, the comparative composition described above was applied to said nail so as to form a base coat.

After application of the base coating, one or more coats of the coloured composition previously described, in the case two coats, was (were) applied to the base coating. After the application of each coat, the nail is placed under a 36 W UV lamp for 3 minutes in order to crosslink the composition so as to form a coloured coating in the form of a film.

After application of the coloured coating and photo-crosslinking of this coating in the form of a film, the top composition previously described is then applied to this coloured coating in the form of one or more coats, in the case a single coat. After the application of this coat, the nail is placed under a 36 W UV lamp for 3 minutes in order to crosslink the composition so as to form a top coating in the form of a film.

After having crosslinked the final coat, the surface is cleaned with cotton wool soaked in isopropanol in order to remove the tacky coat.

The wear property performance levels on the nail, of the compositions according to the invention and also of a comparative composition, are evaluated. Chipping of the varnish, wearing of the varnish and also loss of gloss of the varnish are thus observed for the comparative composition.

In the two embodiments according to the invention evaluated, each including a base coating comprising a photo-crosslinkable composition in accordance with the invention, a varnish exhibiting a good wear property on the nail is thus obtained. To do this, the wear property was assessed by simple observation with the naked eye after 14 days following its application.

It is thus noted that the wear property of the compositions according to the invention comprising a (poly)urethane poly(ALK)acrylate compound comprising a (poly)oxyalkylene unit is better for a period of 5 days, preferably for a period of 10 days and even more preferentially for a period of 14 days compared with a comparative composition comprising a (poly)urethane poly(ALK)acrylate compound free of (poly)oxyalkylene unit.

This wear performance is produced with only a very slight roughening of the nail before application of said compositions, making it possible to avoid the conventional invasive method of attaching a photo-crosslinkable composition to the nails by sanding the surface of the nail, while at the same time preserving performance levels equivalent to or even better than products currently on the market.

Furthermore, the ingredients used in the compositions according to the invention make it possible to have, after photo-crosslinking of the film, an extremely low content of extractable compounds comprising reactive (meth)acrylate functions with potentially sensitising effects.

The varnish can then be completely removed after having been in contact with acetone for 15 minutes, this time again therefore without a conventional invasive method using a metal tool, and electric sander, or an abrasive file by rubbing against the surface of the made-up nail in order to remove the composition.

Throughout the application, the wording "comprising one" or "including one" means "comprising at least one" or "including at least one", unless otherwise specified.

## Claims

1. Photo-crosslinkable cosmetic composition in particular for coating a nail, and more particularly for making up a nail, comprising, in a physiologically acceptable medium:

- at least one photo-crosslinkable compound a) corresponding to formula (I-1) below:

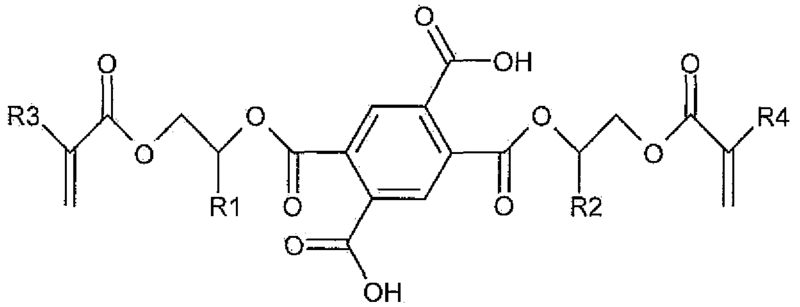

(I-1)

in which formula (I-1):

- R3 and R4, which may be identical or different, represent a hydrogen atom or a methyl group ;
- R1 and R2, which may be identical or different, represent a hydrogen atom, a methyl group or a group of formula (II-1) below:

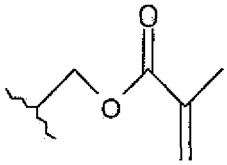

(II-1)

- at least one photo-crosslinkable compound b) corresponding to formula (XI) below:

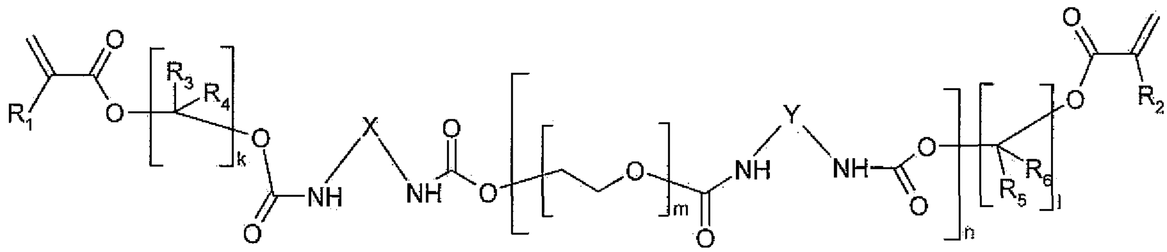

**Formula (XI)**

in which formula (XI):

- $R_1$, $R_2$, $R_3$, $R_4$, $R_5$, and $R_6$, which may be identical or different, represent a hydrogen atom or a $C_1$-$C_{10}$ alkyl chain, preferably a hydrogen atom or a methyl group,
- k and l, which may be identical or different, are between 1 and 10, preferably equal to 2,
- m is between 1 and 100, preferably between 5 and 50, preferably between approximately 8 and 10,
- n is between 1 and 10, preferably equal to 1,
- X and Y, which may be identical or different, represent a $C_1$-$C_{20}$ alkyl or cycloalkyl group,

- at least one film-forming polymer present in a total content strictly less than 30% by weight, relative to the total weight of the solids of the composition.

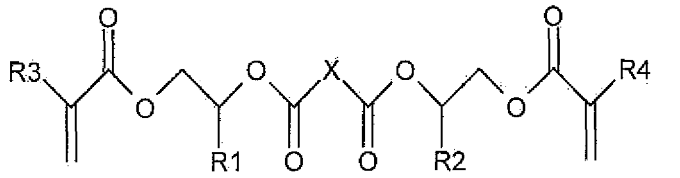

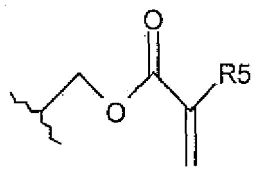

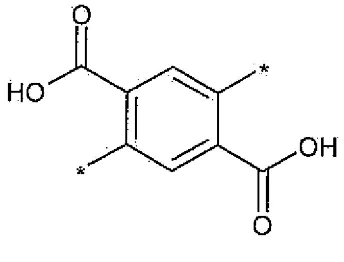

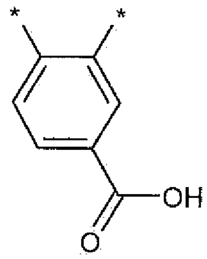

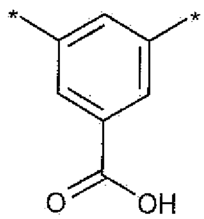

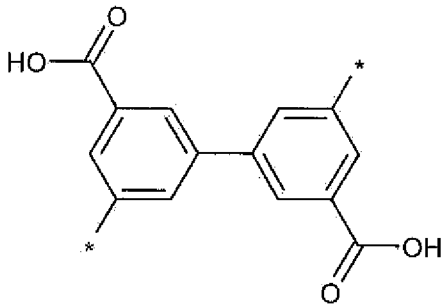

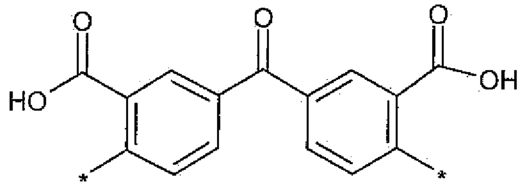

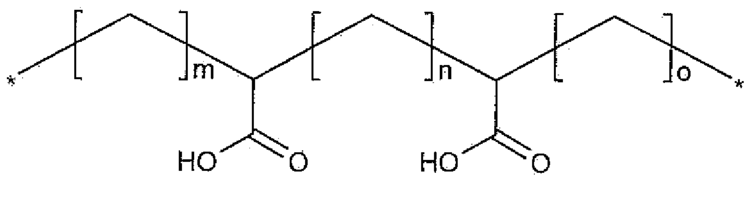

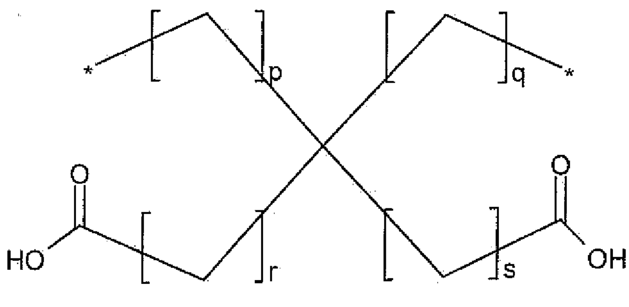

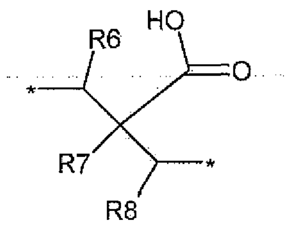

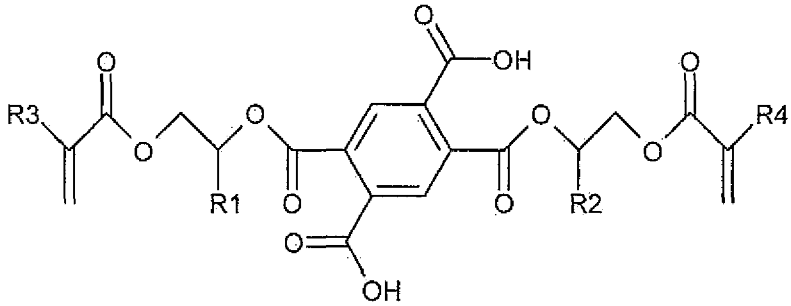

2. Composition according to Claim 1, in which $R_3$ and $R_4$ of formula (I-1) are methyl groups.

3. Composition according to Claim 1 or 2, in which R1 and R2 of formula (I-1) represent a hydrogen atom.

4. Composition according to any one of the preceding claims, in which the compound(s) a), corresponding to formula (I-1), is (are) present in a content greater than or equal to 10% by weight, relative to the total weight of the solids of the composition, preferably in a content ranging from 10% to 25% by weight, and more particularly between 15% and 20% by weight, relative to the total weight of the solids of the composition.

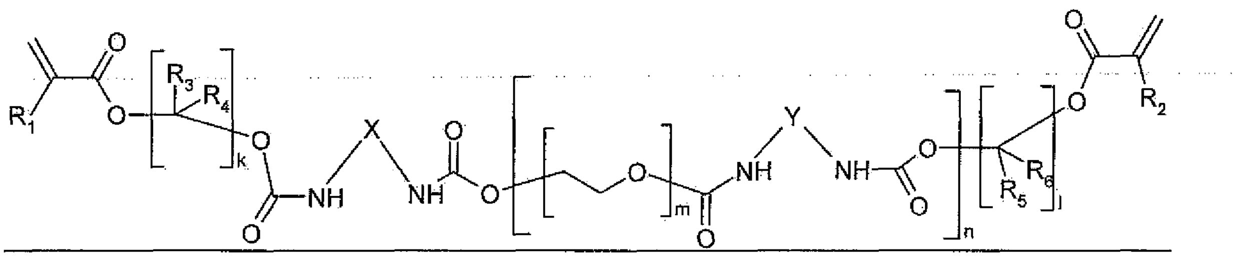

5. Composition according to any one of the preceding claims, in which the compound(s) b) corresponding to formula (XI), is (are) present in a total content greater than or equal to 20% by weight, relative to the total weight of solids of the composition, in particular between 25% and 50% by weight, and more particularly between 30% and 50% by weight, relative to the total weight of the solids of the composition.

6. Composition according to any one of the preceding claims, in which the film-forming polymer(s) is (are) chosen from the group consisting of poly(meth)acrylates, polysaccharides and derivatives, and a mixture thereof, preferably a mixture thereof.

7. Composition according to any one of the preceding claims, in which the film-forming polymer(s) comprise(s) at least one poly(meth)acrylate corresponding to formula (XII) below:

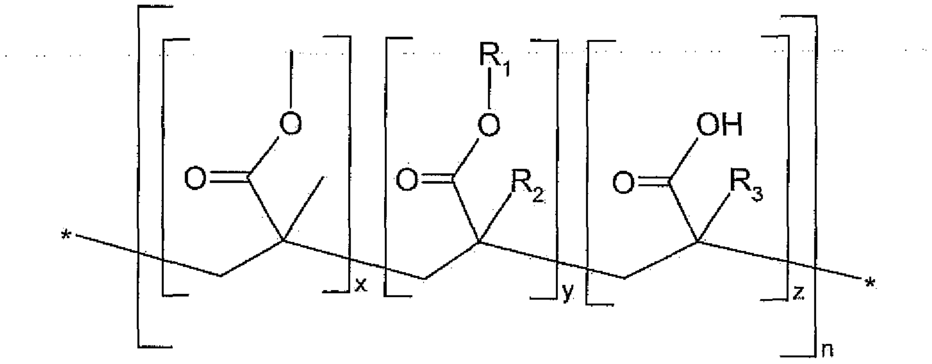

Formula (XII)

in which formula (XII):

- $R_1$, $R_2$ and $R_3$, which may be identical or different, represent a hydrogen atom or a $C_1$-$C_{10}$ alkyl group, $R_1$ preferably representing a $C_4$-$C_{10}$ alkyl group, and $R_2$ and $R_3$ preferably representing a hydrogen atom or a methyl group,
- x and y, which may be identical or different, represent an integer between 1 and 100,
- z represents an integer between 0 and100,
- n represents an integer between 1 and 1000.

**8.** Composition according to any one of the preceding claims, in which the film-forming polymer(s) comprise(s) at least one polysaccharide or polysaccharide derivative chosen from nitrocellulose and ethers and esters of polysaccharides, in particular of $C_2$-$C_4$, in particular from cellulose acetobutyrates, cellulose acetopropionates, ethylcelluloses, ethyl guars, and mixtures thereof, more preferentially chosen from nitrocellulose.

**9.** Composition according to any one of the preceding claims, in which the film-forming polymer(s) is (are) present in a total content greater than or equal to 20% by weight, relative to the total weight of solids of the composition, preferably ranging from 25% to 30% by weight, relative to the total weight of the solids of the composition.

**10.** Composition according to any one of the preceding claims, comprising at least one volatile solvent, preferably at least one polar volatile solvent advantageously chosen from the group consisting of $C_3$-$C_6$ esters and ketones and mixtures thereof.

**11.** Composition according to the preceding claim, in which the volatile solvent(s) is (are) present in a total content greater than or equal to 30% by weight, relative to the total weight of the composition, in particular ranging from 50% to 70%, relative to the total weight of the composition.

**12.** Composition according to any one of the preceding claims, comprising at least one photoinitiator, the photoinitiator preferably being chosen from the group consisting of $\alpha$-hydroxy ketones, $\alpha$-amino ketones, aromatic ketones preferably combined with a hydrogen-donating compound, aromatic $\alpha$-diketones and acylphosphine oxides, and mixtures thereof, advantageously from the group consisting of acylphosphine oxides.

**13.** Composition according to any one of the preceding claims, also comprising at least one (meth)acrylate monomer, preferably in a content less than or equal to 10% by weight relative to the total weight of the solids of the composition.

**14.** Composition according to any one of Claims 1 to **13, characterized in that** it is transparent.

**15.** Method for coating the nails and/or false nails, in particular for making up and/or caring for the nails and/or false nails, comprising at least the following steps:

A) application, to a nail or a false nail, of a composition according to any one of Claims 1 to **14,** via which a coating consisting of at least one coat of said photo-crosslinkable composition is deposited, and
B) exposure of the coated nail or false nail obtained at the end of step A) to a UV or visible light radiation.

**16.** Method for coating the nails and/or false nails, in particular for making up and/or caring for the nails and/or false nails, comprising at least the following steps:

A) application, to a nail or a false nail, of a first composition according to any ne of Claims 1 to **14,** via which a first coating consisting of at least one coat of said photo-crosslinkable composition is deposited, this first coating being applied directly in contact with the nail or the false nail, and
B) exposure of the coated nail or false nail obtained at the end of step A) to a UV or visible light radiation,
C) application, to the first coating resulting from step A) and B), of a second composition, distinct from the first composition, via which a second coating consisting of at least one coat of said second composition is deposited,
D) exposure of the coated nail or false nail obtained at the end of step C) to a UV or visible light radiation.

**17.** Method for coating the nails and/or false nails, in particular for making up and/or caring for the nails and/or false nails, comprising at least the following steps:

A) application, to a nail or a false nail, of a first composition according to any one of Claims 1 to 14, via which a first coating consisting of at least one coat of said photo-crosslinkable composition is deposited, this first coating being applied directly in contact with the nail or the false nail, and
B) exposure of the coated nail or false nail obtained at the end of step A) to a UV or visible light radiation,
C) application, to the first coating resulting from step A) and B), of a second composition, distinct from the first composition, via which a second coating consisting of at least one coat of said second composition is deposited,
D) exposure of the coated nail or false nail obtained at the end of step C) to a UV or visible light radiation,
E) application, to the second coating resulting from step C) and D), of a third composition, distinct from the first composition from the second composition, via which a third coating consisting of at least one coat of said third composition is deposited,
F) exposure of the coated nail or false nail obtained at the end of step E) to a UV or visible light radiation.

**18.** Method for coating the nails and/or false nails, in particular for making up and/or caring for the nails and/or false nails, comprising at least the following steps:

A) application, to a nail or a false nail, of a first composition according to any one of Claims 1 to 14, via which a first coating consisting of at least one coat of said photo-crosslinkable composition is deposited,
B) exposure of the coated nail or false nail obtained at the end of step A) to a UV or visible light radiation,
C) application, to the first coating resulting from step A) and B), of a second composition, distinct from the first composition, via which a second coating consisting of at least one coat of said second composition is deposited, the second composition comprising:

- at least one urethane (meth)acrylate compound corresponding to formula (XIII) below:

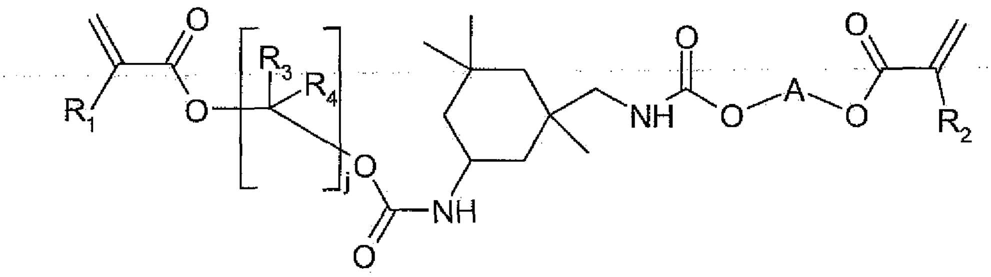

Formula (XIII)

in which formula XIII:

- $R_1$, $R_2$, $R_3$ and $R_4$, which may be identical or different, represent a hydrogen atom or a $C_1$-$C_{10}$ alkyl chain, preferably a hydrogen atom or a methyl group,
- j is between 1 and 10, preferably equal to 2,
- A represents a $C_1$-$C_{10}$ alkyl group, or a polyurethane comprising from 2 to 20 carbamate units.
- at least one photo-crosslinkable compound b) corresponding to formula (XI) below:

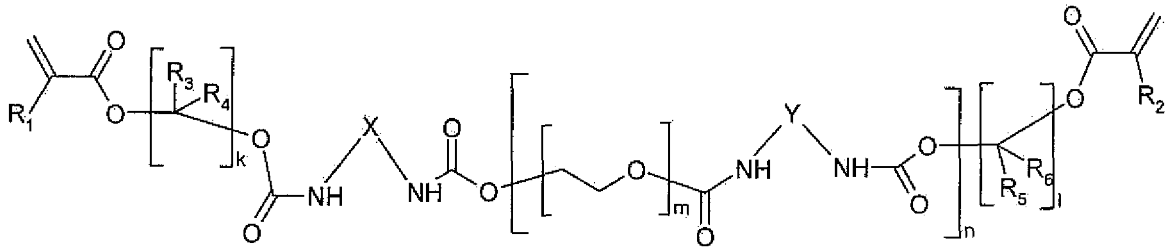

Formula (XI)

in which formula (XI):

- $R_1$, $R_2$, $R_3$, $R_4$, $R_5$, and $R_6$, which may be identical or different, represent a hydrogen atom or a $C_1$-$C_{10}$ alkyl chain, preferably a hydrogen atom or a methyl group,
- k and l, which may be identical or different, are between 1 and 10, preferably equal to 2,
- m is between 1 and 100, preferably between 5 and 50, preferably between approximately 8 and 10,
- n is between 1 and 10, preferably equal to 1,
- X and Y, which may be identical or different, represent a $C_1$-$C_{20}$ alkyl or cycloalkyl group,
- at least one photoinitiator, preferably chosen from the group consisting of $\alpha$-hydroxy ketones, $\alpha$-amino ketones, aromatic ketones preferably combined with a hydrogen-donating compound, aromatic $\alpha$-diketones and acylphosphine oxides, and mixtures thereof, advantageously from the group consisting of acylphosphine oxides,
- preferably at least one monofunctional (meth)acrylate monomer, preferably tetrahydrofurfuryl methacrylate (THFMA),
- optionally at least one colouring agent, in particular at least one pigment,

D) exposure of the coated nail or false nail obtained at the end of step C) to a UV or visible light radiation,
E) optionally, application, to the second coating resulting from step C) and D), of a third composition, via which a third coating consisting of at least one coat of said third composition is deposited, the third composition comprising:

- at least one urethane (meth)acrylate compound corresponding to formula (XIII) below:

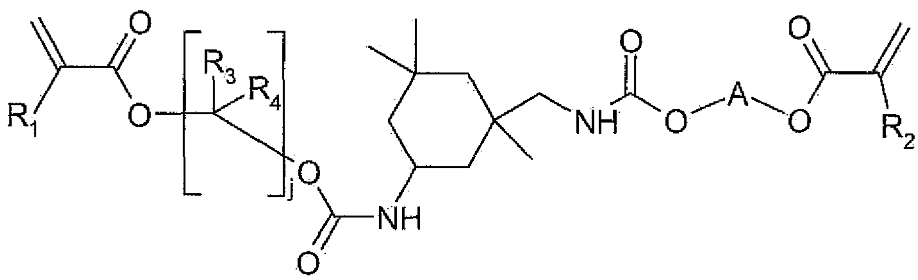

Formula (XIII)

in which formula (XIII):

- $R_1$, $R_2$, $R_3$ and $R_4$, which may be identical or different, represent a hydrogen atom or a $C_1$-$C_{10}$ alkyl chain, preferably a hydrogen atom or a methyl group,
- j is between 1 and 10, preferably equal to 2,
- A represents a $C_1$-$C_{10}$ alkyl group, or a polyurethane comprising from 2 to 20 carbamate units.
- at least one photo-crosslinkable compound b) corresponding to formula (XI) below:

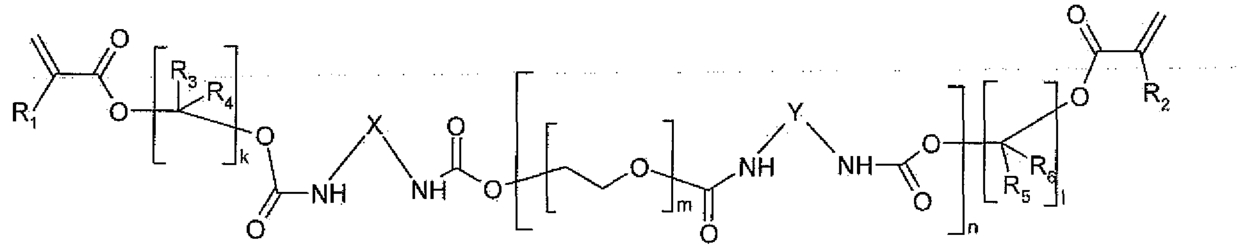

Formula (XI)

in which formula (XI):

- $R_1$, $R_2$, $R_3$, $R_4$, $R_5$, and $R_6$, which may be identical or different, represent a hydrogen atom or a $C_1$-$C_{10}$ alkyl chain, preferably a hydrogen atom or a methyl group,
- with k and l, which may be identical or different, between 1 and 10, preferably equal to 2,
- with m between 1 and 100, preferably between 5 and 50, preferably between approximately 8 and 10,
- with n between 1 and 10, preferably equal to 1,
- with X and Y, which may be identical or different, representing a $C_1$-$C_{20}$ alkyl or cycloalkyl group,
- at least one photoinitiator, preferably chosen from the group consisting of $\alpha$-hydroxy ketones, $\alpha$-amino ketones, aromatic ketones preferably combined with a hydrogen-donating compound, aromatic $\alpha$-diketones and acylphosphine oxides, and mixtures thereof, advantageously from the group consisting of acylphosphine oxides,
- preferably at least one monofunctional (meth)acrylate monomer, preferably tetrahydrofurfuryl methacrylate (THFMA),

F) exposure of the coated nail or false nail obtained at the end of step E) to a UV or visible light radiation.

**19.** Method for coating the nails and/or false nails according to Claim **17 or 18,** in which, when a third coating of a third composition is applied, the second composition applied as second coating comprises at least one colouring agent.

**20.** Method for coating the nails and/or false nails according to Claim **17,18 or 19,** in which steps C) and D) are repeated in total twice, the second composition being applied a first time and exposed to a UV or visible light radiation, then being applied a second time and exposed to a UV or visible light radiation, steps A) and B), like E) and F), preferably being respectively carried out only once.

**Patentansprüche**

**1.** Photovernetzbare kosmetische Zusammensetzung, insbesondere zum Beschichten eines Nagels und spezieller zum Zurechtmachen eines Nagels, umfassend in einem physiologisch unbedenklichen Medium:

- mindestens eine photovernetzbare Verbindung a), die der nachstehenden Formel (I-1) entspricht:

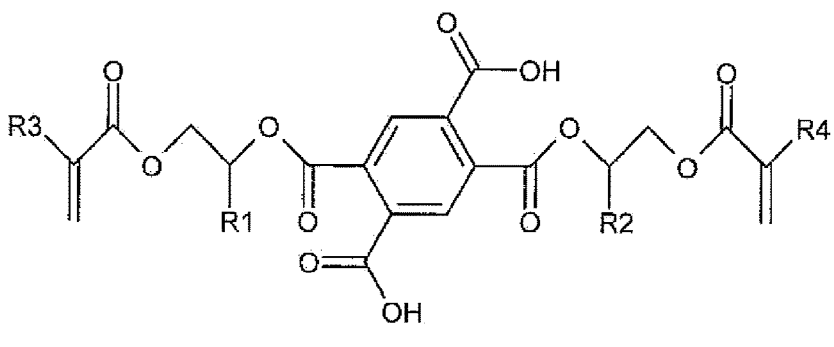

**(I-1)**

wobei in der Formel (I-1):

- R3 und R4, die gleich oder verschieden sein können, für ein Wasserstoffatom oder eine Methylgruppe stehen;
- R1 und R2, die gleich oder verschieden sein können, für ein Wasserstoffatom, eine Methylgruppe oder eine Gruppe der nachstehenden Formel (II-1) stehen:

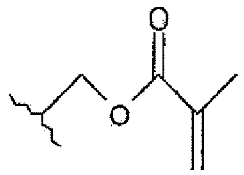

(II-1)

- mindestens eine photovernetzbare Verbindung b), die der nachstehenden Formel (XI) entspricht:

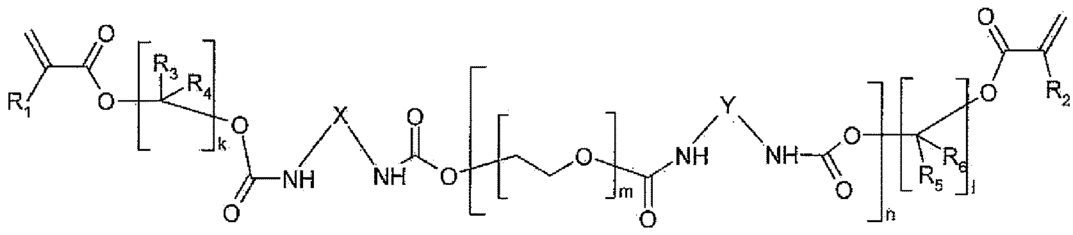

Formel (XI)

wobei in der Formel (XI):

- $R_1$, $R_2$, $R_3$, $R_4$, $R_5$ und $R_6$, die gleich oder verschieden sein können, für ein Wasserstoffatom oder eine $C_1$-$C_{10}$-Alkylkette, vorzugsweise ein Wasserstoffatom oder eine Methylgruppe, stehen,
- k und l, die gleich oder verschieden sein können, zwischen 1 und 10 liegen und vorzugsweise gleich 2 sind,
- m zwischen 1 und 100, vorzugsweise zwischen 5 und 50, vorzugsweise zwischen ungefähr 8 und 10, liegt,
- n zwischen 1 und 10 liegt und vorzugsweise gleich 1 ist,
- X und Y, die gleich oder verschieden sein können, für eine $C_1$-$C_{20}$-Alkyl- oder Cycloalkylgruppe stehen,
- mindestens ein filmbildendes Polymer, das in einem Gesamtgehalt von strikt weniger als 30 Gew.-%, bezogen auf das Gesamtfeststoffgewicht der Zusammensetzung, vorliegt.

2. Zusammensetzung nach Anspruch 1, wobei R3 und R4 von Formel (I-1) Methylgruppen sind.

3. Zusammensetzung nach Anspruch 1 oder 2, wobei R1 und R2 von Formel (I-1) für ein Wasserstoffatom stehen.

4. Zusammensetzung nach einem der vorhergehenden Ansprüche, wobei die Verbindung bzw. Verbindungen a), die der Formel (I-1) entspricht bzw. entsprechen, in einem Gehalt größer oder gleich 10 Gew.-%, bezogen auf das Gesamtgewicht der Feststoffe der Zusammensetzung, vorzugsweise in einem Gehalt im Bereich von 10 bis 25 Gew.-% und spezieller zwischen 15 und 20 Gew.-%, bezogen auf das Gesamtgewicht der Feststoffe der Zusammensetzung, vorliegt bzw. vorliegen.

5. Zusammensetzung nach einem der vorhergehenden Ansprüche, wobei die Verbindung bzw. Verbindungen b), die der Formel (XI) entspricht bzw. entsprechen, in einem Gesamtgehalt größer oder gleich 20 Gew.-%, bezogen auf das Gesamtgewicht der Feststoffe der Zusammensetzung, insbesondere zwischen 25 und 50 Gew.-% und spezieller zwischen 30 und 50 Gew.-%, bezogen auf das Gesamtgewicht der Feststoffe der Zusammensetzung, vorliegt bzw. vorliegen.

6. Zusammensetzung nach einem der vorhergehenden Ansprüche, wobei das filmbildende Polymer bzw. die filmbildenden Polymere aus der Gruppe bestehend aus Poly(meth)acrylaten, Polysacchariden und Derivaten und einer Mischung davon, vorzugsweise einer Mischung davon, ausgewählt ist bzw. sind.

7. Zusammensetzung nach einem der vorhergehenden Ansprüche, wobei das filmbildende Polymer bzw. die filmbildenden Polymere mindestens ein Poly(meth)-acrylat der nachstehenden Formel (XII) umfasst bzw. umfassen:

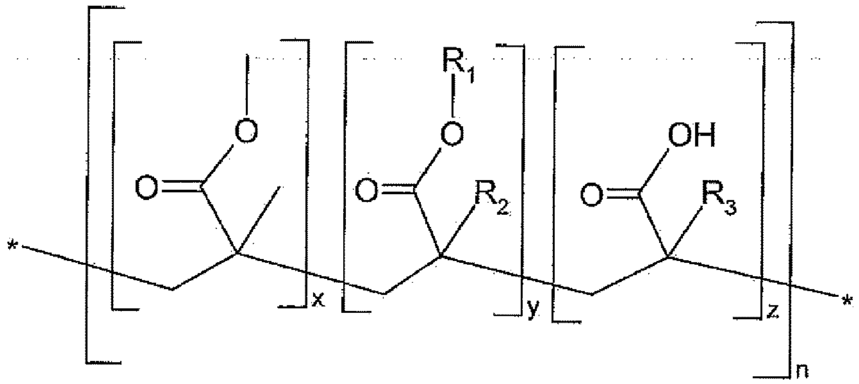

Formel (XII)

wobei in der Formel (XII):

- $R_1$, $R_2$ und $R_3$, die gleich oder verschieden sein können, für ein Wasserstoffatom oder eine $C_1$-$C_{10}$-Alkylgruppe stehen, wobei $R_1$ vorzugsweise für eine $C_4$-$C_{10}$-Alkylgruppe steht und $R_2$ und $R_3$ vorzugsweise für ein Wasserstoffatom oder eine Methylgruppe stehen,
- x und y, die gleich oder verschieden sein können, für eine ganze Zahl zwischen 1 und 100 stehen,
- z für eine ganze Zahl zwischen 0 und 100 steht,
- n für eine ganze Zahl zwischen 1 und 1000 steht.

**8.** Zusammensetzung nach einem der vorhergehenden Ansprüche, wobei das filmbildende Polymer bzw. die filmbildenden Polymere mindestens ein Polysaccharid oder Polysaccharid-Derivat, das aus Nitrocellulose und Ethern und -Estern von Polysacchariden, insbesondere $C_2$-$C_4$-Ethern und -Estern von Polysacchariden, insbesondere aus Celluloseacetobutyraten, Celluloseacetopropionaten, Ethylcellulosen, Ethylguarverbindungen und Mischungen davon, weiter bevorzugt aus Nitrocellulose, ausgewählt ist, umfasst bzw. umfassen.

**9.** Zusammensetzung nach einem der vorhergehenden Ansprüche, wobei das filmbildende Polymer bzw. die filmbildenden Polymere in einem Gesamtgehalt größer oder gleich 20 Gew.-%, bezogen auf das Gesamtgewicht der Feststoffe der Zusammensetzung, vorzugsweise im Bereich von 25 bis 30 Gew.-%, bezogen auf das Gesamtgewicht der Feststoffe der Zusammensetzung, vorliegt bzw. vorliegen.

**10.** Zusammensetzung nach einem der vorhergehenden Ansprüche, umfassend mindestens ein flüchtiges Lösungsmittel, vorzugsweise mindestens ein polares flüchtiges Lösungsmittel, das vorteilhafterweise aus der Gruppe bestehend aus $C_3$-$C_6$-Estern und -Ketonen und Mischungen davon ausgewählt ist.

**11.** Zusammensetzung nach dem vorhergehenden Anspruch, wobei das flüchtige Lösungsmittel bzw. die flüchtigen Lösungsmittel in einem Gesamtgehalt größer oder gleich 30 Gew.-%, bezogen auf das Gesamtgewicht der Zusammensetzung, insbesondere im Bereich von 50 % bis 70 %, bezogen auf das Gesamtgewicht der Zusammensetzung, vorliegt bzw. vorliegen.

**12.** Zusammensetzung nach einem der vorhergehenden Ansprüche, umfassend mindestens einen Photoinitiator, wobei der Photoinitiator vorzugsweise aus der Gruppe bestehend aus $\alpha$-Hydroxyketonen, $\alpha$-Aminoketonen, aromatischen Ketonen, vorzugsweise in Kombination mit einem Wasserstoffdonor, aromatischen $\alpha$-Diketonen und Acylphosphinoxiden und Mischungen davon, vorteilhafterweise aus der Gruppe bestehend aus Acylphosphinoxiden, ausgewählt ist.

**13.** Zusammensetzung nach einem der vorhergehenden Ansprüche, außerdem umfassend mindestens ein (Meth)acrylat-Monomer, vorzugsweise in einem Gehalt kleiner oder gleich 10 Gew.-%, bezogen auf das Gesamtgewicht der Feststoffe der Zusammensetzung.

**14.** Zusammensetzung nach einem der Ansprüche 1 bis 13, **dadurch gekennzeichnet, dass** sie transparent ist.

**15.** Verfahren zum Beschichten der Nägel und/oder künstlichen Nägel, insbesondere zum Zurechtmachen und/oder

Pflegen der Nägel und/oder künstlichen Nägel, das mindestens die folgenden Schritte umfasst:

A) Aufbringen einer Zusammensetzung nach einem der Ansprüche 1 bis 14 auf einen Nagel oder einen künstlichen Nagel, wodurch eine Beschichtung, die aus mindestens einer Schicht der photovernetzbaren Zusammensetzung besteht, abgeschieden wird, und
B) Einwirkenlassen von UV- oder sichtbarer Lichtstrahlung auf den am Ende von Schritt A) erhaltenen beschichteten Nagel oder künstlichen Nagel.

**16.** Verfahren zum Beschichten der Nägel und/oder künstlichen Nägel, insbesondere zum Zurechtmachen und/oder Pflegen der Nägel und/oder künstlichen Nägel, das mindestens die folgenden Schritte umfasst:

A) Aufbringen einer ersten Zusammensetzung nach einem der Ansprüche 1 bis 14 auf einen Nagel oder einen künstlichen Nagel, wodurch eine erste Beschichtung, die aus mindestens einer Schicht der photovernetzbaren Zusammensetzung besteht, abgeschieden wird, wobei diese erste Beschichtung direkt in Kontakt mit dem Nagel oder dem künstlichen Nagel aufgebracht wird, und
B) Einwirkenlassen von UV- oder sichtbarer Lichtstrahlung auf den am Ende von Schritt A) erhaltenen beschichteten Nagel oder künstlichen Nagel,
C) Aufbringen einer zweiten Zusammensetzung, die von der ersten Zusammensetzung verschieden ist, auf die aus Schritt A) und B) resultierende erste Beschichtung, wodurch eine zweite Beschichtung, die aus mindestens einer Schicht der zweiten Zusammensetzung besteht, abgeschieden wird,
D) Einwirkenlassen von UV- oder sichtbarer Lichtstrahlung auf den am Ende von Schritt C) erhaltenen beschichteten Nagel oder künstlichen Nagel.

**17.** Verfahren zum Beschichten der Nägel und/oder künstlichen Nägel, insbesondere zum Zurechtmachen und/oder Pflegen der Nägel und/oder künstlichen Nägel, das mindestens die folgenden Schritte umfasst:

A) Aufbringen einer ersten Zusammensetzung nach einem der Ansprüche 1 bis 14 auf einen Nagel oder einen künstlichen Nagel, wodurch eine erste Beschichtung, die aus mindestens einer Schicht der photovernetzbaren Zusammensetzung besteht, abgeschieden wird, wobei diese erste Beschichtung direkt in Kontakt mit dem Nagel oder dem künstlichen Nagel aufgebracht wird, und
B) Einwirkenlassen von UV- oder sichtbarer Lichtstrahlung auf den am Ende von Schritt A) erhaltenen beschichteten Nagel oder künstlichen Nagel,
C) Aufbringen einer zweiten Zusammensetzung, die von der ersten Zusammensetzung verschieden ist, auf die aus Schritt A) und B) resultierende erste Beschichtung, wodurch eine zweite Beschichtung, die aus mindestens einer Schicht der zweiten Zusammensetzung besteht, abgeschieden wird,
D) Einwirkenlassen von UV- oder sichtbarer Lichtstrahlung auf den am Ende von Schritt C) erhaltenen beschichteten Nagel oder künstlichen Nagel,
E) Aufbringen einer dritten Zusammensetzung, die von der ersten Zusammensetzung und von der zweiten Zusammensetzung verschieden ist, auf die aus Schritt C) und D) resultierende zweite Beschichtung, wodurch eine dritte Beschichtung, die aus mindestens einer Schicht der dritten Zusammensetzung besteht, abgeschieden wird,
F) Einwirkenlassen von UV- oder sichtbarer Lichtstrahlung auf den am Ende von Schritt E) erhaltenen beschichteten Nagel oder künstlichen Nagel.

**18.** Verfahren zum Beschichten der Nägel und/oder künstlichen Nägel, insbesondere zum Zurechtmachen und/oder Pflegen der Nägel und/oder künstlichen Nägel, das mindestens die folgenden Schritte umfasst:

A) Aufbringen einer ersten Zusammensetzung nach einem der Ansprüche 1 bis 14 auf einen Nagel oder einen künstlichen Nagel, wodurch eine erste Beschichtung, die aus mindestens einer Schicht der photovernetzbaren Zusammensetzung besteht, abgeschieden wird,
B) Einwirkenlassen von UV- oder sichtbarer Lichtstrahlung auf den am Ende von Schritt A) erhaltenen beschichteten Nagel oder künstlichen Nagel,
C) Aufbringen einer zweiten Zusammensetzung, die von der ersten Zusammensetzung verschieden ist, auf die aus Schritt A) und B) resultierende erste Beschichtung, wodurch eine zweite Beschichtung, die aus mindestens einer Schicht der zweiten Zusammensetzung besteht, abgeschieden wird, wobei die zweite Zusammensetzung Folgendes umfasst:

- mindestens eine Urethan(meth)acrylat-Verbindung, die der nachstehenden Formel (XIII) entspricht:

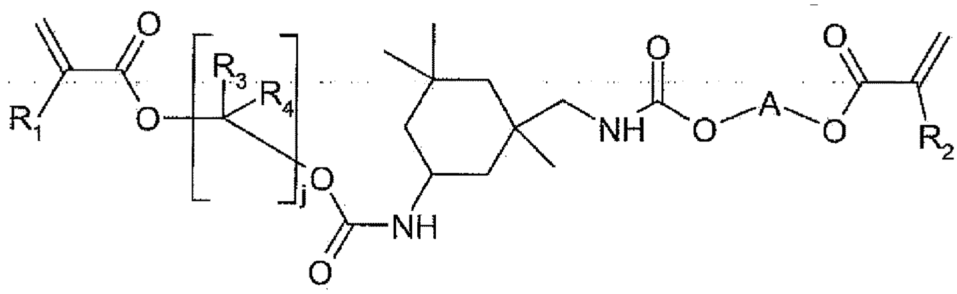

Formel (XIII)

wobei in der Formel (XIII):

- $R_1$, $R_2$, $R_3$ und $R_4$, die gleich oder verschieden sein können, für ein Wasserstoffatom oder eine $C_1$-$C_{10}$-Alkylkette, vorzugsweise ein Wasserstoffatom oder eine Methylgruppe, stehen,
- j zwischen 1 und 10 liegt und vorzugsweise gleich 2 ist,
- A für eine $C_1$-$C_{10}$-Alkylgruppe oder ein Polyurethan mit 2 bis 20 Carbamat-Einheiten steht,
- mindestens eine photovernetzbare Verbindung b), die der nachstehenden Formel (XI) entspricht:

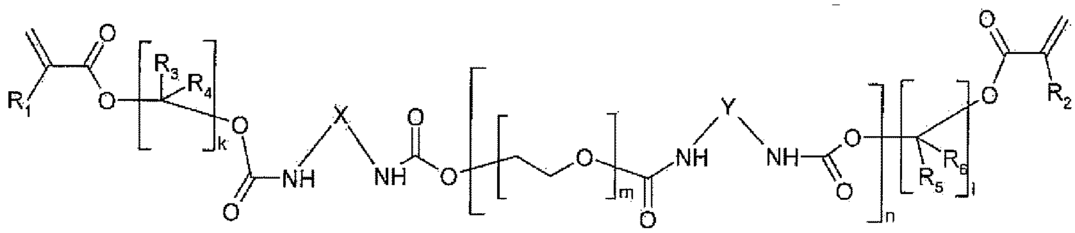

Formel (XI)

wobei in der Formel (XI):

- $R_1$, $R_2$, $R_3$, $R_4$, $R_5$ und $R_6$, die gleich oder verschieden sein können, für ein Wasserstoffatom oder eine $C_1$-$C_{10}$-Alkylkette, vorzugsweise ein Wasserstoffatom oder eine Methylgruppe, stehen,
- k und l, die gleich oder verschieden sein können, zwischen 1 und 10 liegen und vorzugsweise gleich 2 sind,
- m zwischen 1 und 100, vorzugsweise zwischen 5 und 50, vorzugsweise zwischen ungefähr 8 und 10, liegt,
- n zwischen 1 und 10 liegt und vorzugsweise gleich 1 ist,
- X und Y, die gleich oder verschieden sein können, für eine $C_1$-$C_{20}$-Alkyl- oder Cycloalkylgruppe stehen,
- mindestens einen Photoinitiator, der vorzugsweise aus der Gruppe bestehend aus $\alpha$-Hydroxyketonen, $\alpha$-Aminoketonen, aromatischen Ketonen, vorzugsweise in Kombination mit einem Wasserstoffdonor, aromatischen $\alpha$-Diketonen und Acylphosphinoxiden und Mischungen davon, vorteilhafterweise aus der Gruppe bestehend aus Acylphosphinoxiden, ausgewählt ist,
- vorzugsweise mindestens ein monofunktionelles (Meth)acrylat-Monomer, vorzugsweise Tetrahydrofurfurylmethacrylat (THFMA),
- gegebenenfalls mindestens ein Farbmittel, insbesondere mindestens ein Pigment,

D) Einwirkenlassen von UV- oder sichtbarer Lichtstrahlung auf den am Ende von Schritt C) erhaltenen beschichteten Nagel oder künstlichen Nagel,
E) gegebenenfalls Aufbringen einer dritten Zusammensetzung auf die aus Schritt C) und D) resultierende zweite Beschichtung, wodurch eine dritte Beschichtung, die aus mindestens einer Schicht der dritten Zusammensetzung besteht, abgeschieden wird,, wobei die dritte Zusammensetzung Folgendes umfasst:

- mindestens eine Urethan (meth) acrylat-Verbindung, die der nachstehenden Formel (XIII) entspricht:

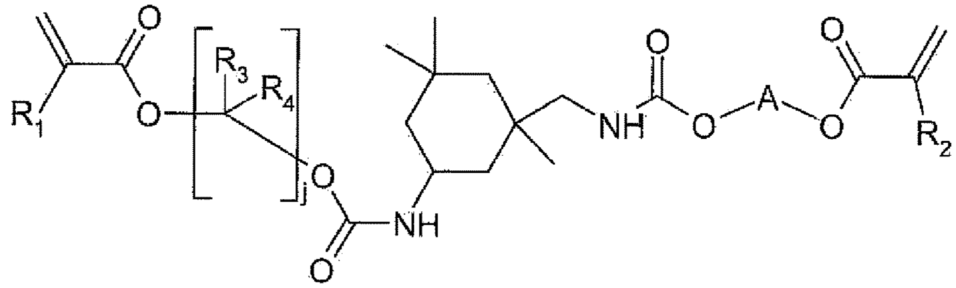

Formel (XIII)

wobei in der Formel (XIII):

- $R_1$, $R_2$, $R_3$ und $R_4$, die gleich oder verschieden sein können, für ein Wasserstoffatom oder eine $C_1$-$C_{10}$-Alkylkette, vorzugsweise ein Wasserstoffatom oder eine Methylgruppe, stehen,
- j zwischen 1 und 10 liegt und vorzugsweise gleich 2 ist,
- A für eine $C_1$-$C_{10}$-Alkylgruppe oder ein Polyurethan mit 2 bis 20 Carbamat-Einheiten steht,
- mindestens eine photovernetzbare Verbindung b), die der nachstehenden Formel (XI) entspricht:

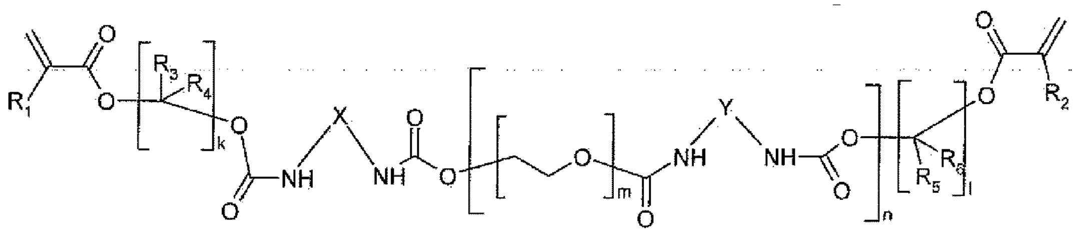

Formel (XI)

wobei in der Formel (XI):

- $R_1$, $R_2$, $R_3$, $R_4$, $R_5$ und $R_6$, die gleich oder verschieden sein können, für ein Wasserstoffatom oder eine $C_1$-$C_{10}$-Alkylkette, vorzugsweise ein Wasserstoffatom oder eine Methylgruppe, stehen,
- k und l, die gleich oder verschieden sein können, zwischen 1 und 10 liegen und vorzugsweise gleich 2 sind,
- m zwischen 1 und 100, vorzugsweise zwischen 5 und 50, vorzugsweise zwischen ungefähr 8 und 10, liegt,
- n zwischen 1 und 10 liegt und vorzugsweise gleich 1 ist,
- X und Y, die gleich oder verschieden sein können, für eine $C_1$-$C_{20}$-Alkyl- oder Cycloalkylgruppe stehen,
- mindestens einen Photoinitiator, der vorzugsweise aus der Gruppe bestehend aus $\alpha$-Hydroxyketonen, $\alpha$-Aminoketonen, aromatischen Ketonen, vorzugsweise in Kombination mit einem Wasserstoffdonor, aromatischen $\alpha$-Diketonen und Acylphosphinoxiden und Mischungen davon, vorteilhafterweise aus der Gruppe bestehend aus Acylphosphinoxiden, ausgewählt ist,
- vorzugsweise mindestens ein monofunktionelles (Meth)acrylat-Monomer, vorzugsweise Tetrahydrofurfurylmethacrylat (THFMA),

F) Einwirkenlassen von UV- oder sichtbarer Lichtstrahlung auf den am Ende von Schritt E) erhaltenen beschichteten Nagel oder künstlichen Nagel.

**19.** Verfahren zum Beschichten der Nägel und/oder künstlichen Nägel nach Anspruch 17 oder 18, bei dem dann, wenn eine dritte Beschichtung einer dritten Zusammensetzung aufgebracht wird, die als zweite Beschichtung aufgebrachte zweite Zusammensetzung mindestens ein Farbmittel umfasst.

**20.** Verfahren zum Beschichten der Nägel und/oder künstlichen Nägel nach Anspruch 17, 18 oder 19, bei dem die Schritte C) und D) insgesamt zweimal wiederholt werden, wobei die zweite Zusammensetzung ein erstes Mal aufgebracht und UV- oder sichtbarer Lichtstrahlung ausgesetzt wird, dann ein zweites Mal aufgebracht und UV-

oder sichtbarer Lichtstrahlung ausgesetzt wird, wobei die Schritte A) und B), wie E) und F), vorzugsweise jeweils nur einmal durchgeführt werden.

## Revendications

**1.** Composition cosmétique photoréticulable, en particulier destinée au revêtement d'un ongle, et plus particulièrement destinée au maquillage d'un ongle, comprenant, dans un milieu physiologiquement acceptable :

- au moins un composé photoréticulable a) répondant à la formule (I-1) ci-dessous :

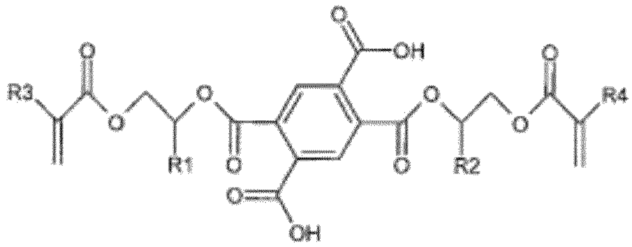

(I-1)

Formule (I-1) dans laquelle :

- R3 et R4, identiques ou différents, représentent un atome d'hydrogène ou un groupe méthyle ;
- R1 et R2, identiques ou différents, représentent un atome d'hydrogène, ou un groupe méthyle, ou un groupe de formule (II-1) suivante :

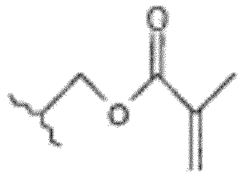

(II-1)

- au moins un composé photoréticulable b) répondant à la formule (XI) ci-dessous :

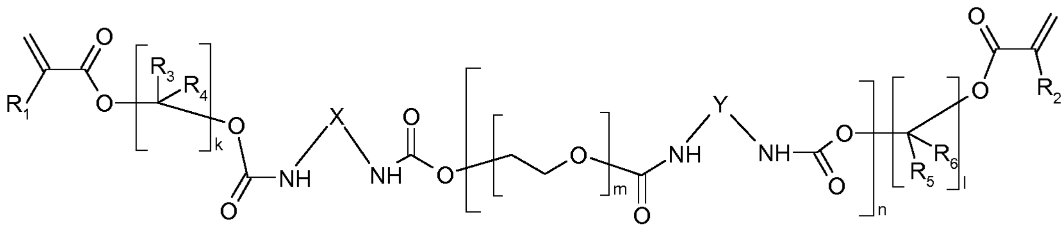

Formule (XI)

Formule (XI) dans laquelle :

$R_1$, $R_2$, $R_3$, $R_4$, $R_5$, et $R_6$, identiques ou différents,
représentent un atome d'hydrogène ou une chaîne alkyle en $C_1$-$C_{10}$, de préférence un atome d'hydrogène ou un groupe méthyle,
k et l, identiques ou différents, sont compris entre 1 et 10, de préférence égaux à 2,
m est compris entre 1 et 100, de préférence compris entre 5 et 50, de préférence compris entre environ 8 et 10,
n est compris entre 1 et 10, de préférence égal à 1,
X et Y, identiques ou différents, représentent un groupe cycloalkyle ou alkyle en $C_1$-$C_{20}$,

- au moins un polymère filmogène présent en une teneur totale strictement inférieure à 30 % en poids, par rapport au poids total de l'extrait sec de la composition.

2. Composition selon la revendication 1, dans laquelle R3 et R4 de la formule (I-1) sont des groupes méthyles.

3. Composition selon la revendication 1 ou 2, dans laquelle R1 et R2 de la formule (I-1) représentent un atome d'hydrogène.

4. Composition selon l'une quelconque des revendications précédentes, dans laquelle le/les composé (s) a), répondant à la formule (I-1), est/sont présent (s) à une teneur supérieure ou égale à 10 % en poids, par rapport au poids total de l'extrait sec de la composition, de préférence à une teneur allant de 10 à 25 % en poids, et plus particulièrement entre 15 et 20 % en poids, par rapport au poids total de l'extrait sec de la composition.

5. Composition selon l'une quelconque des revendications précédentes, dans laquelle le/les compose(s) b), en particulier répondant à la formule (XI), est/sont present (s) à une eneur totale supérieure ou égale à 20 % en poids par rapport au poids total d'extrait sec de la composition, en particulier compris entre 25 et 50 % en poids, et plus particulièrement entre 30 et 50 % en poids, par rapport au poids total de l'extrait sec de la composition.

6. Composition selon l'une quelconque des revendications précédents, dans lawuelle le/les polymère(s) filmogène(s) est/sont choisi(s) dans le groupe constitué des poly(méth)acrylates, des polysaccharides et dérivés, et de leur mélange, de preference leur mélange.

7. Composition selon l'une quelconque des revendications précédentes, dans laquelle le/les polymère(s) filmogène(s) comprend/comprennent au moins un poly(méth)acrylate répondant à la formule (XII) suivante :

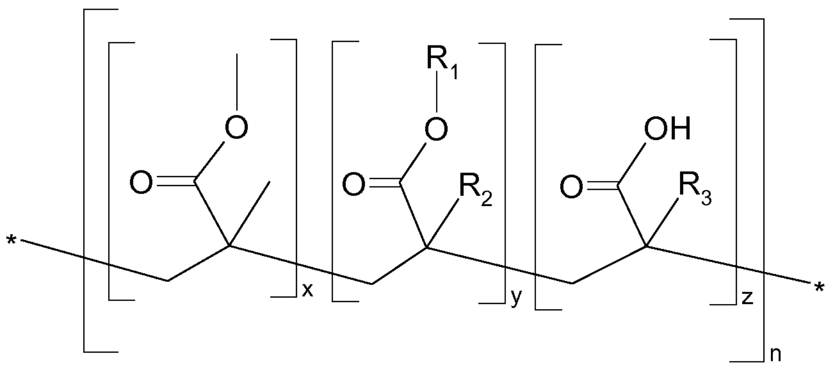

Formule (XII)

Formule (XII) dans laquelle :

$R_1$, $R_2$ et $R_3$, identiques ou différents, représentent un atome d'hydrogène ou un groupe alkyle en $C_1$-$C_{10}$, $R_1$ représentant de préférence un groupe alkyle en $C_4$-$C_{10}$, et $R_2$ et $R_3$ représentent de préférence un atome d'hydrogène ou un groupe méthyle,
x et y, identiques ou différents, représentent un nombre entier compris entre 1 et 100,
z représente un nombre entier compris entre 0 et 100,
n représente un nombre entier compris entre 1 et 1000.

8. Composition selon l'une quelconque des revendications précédentes, dans laquelle le/les polymère(s) filmogène(s) comprend/comprennent au moins un polysaccharide ou dérivé de polysaccharide choisi parmi la nitrocellulose et les éthers et esters de polysaccharides, notamment en $C_2$-$C_4$, notamment parmi les acétobutyrates de cellulose, les acétopropionates de cellulose, les éthylcelluloses, les éthylguars, et leurs mélanges, plus préférentiellement choisi parmi la nitrocellulose.

9. Composition selon l'une quelconque des revendications précédentes, dans laquelle le/les polymère(s) filmogène(s) est/sont présent (s) à une teneur totale supérieure ou égale à 20 % en poids, par rapport au poids total de l'extrait sec de la composition, de préférence allant de 25 à 30 % en poids, par rapport au poids total de l'extrait sec de la composition.

**10.** Composition selon l'une quelconque des revendications précédentes, comprenant au moins un solvant volatil, de préférence au moins un solvant volatil polaire avantageusement choisi dans le groupe constitué des cétones et des esters en $C_3$-$C_6$ et leurs mélanges.

**11.** Composition selon la revendication précédente, dans laquelle le/les solvants volatil(s) est/sont présent(s) à une teneur totale supérieure ou égale à 30 % en poids par rapport au poids total de la composition, en particulier allant de 50 à 70 % par rapport au poids total de la composition.

**12.** Composition selon l'une quelconque des revendications précédentes, comprenant au moins un photoamorceur, le photoamorceur étant de préférence choisi dans le groupe constitué des $\alpha$-hydroxycétones, des $\alpha$-aminocétones, des cétones aromatiques de préférence associées à un composé donneur d'hydrogène, des $\alpha$-dicétones aromatiques et des oxydes d'acylphosphine, et de leurs mélanges, avantageusement dans le groupe constitué des oxydes d'acylphosphine.

**13.** Composition selon l'une quelconque des revendications précédentes, comprenant en outre au moins un monomère (méth)acrylate, de préférence à une teneur inférieure ou égale à 10 % en poids par rapport au poids total de l'extrait sec de la composition.

**14.** Composition selon l'une quelconque des revendications 1 à 13, **caractérisée en ce qu'**elle est transparente.

**15.** Procédé de revêtement des ongles et/ou des faux-ongles, en particulier de maquillage et/ou de soin des ongles et/ou des faux-ongles, comprenant au moins les étapes suivantes :

A) application, sur un ongle ou un faux-ongle, d'une composition selon l'une quelconque des revendications 1 à 14, par laquelle on dépose un revêtement constitué d'au moins une couche de ladite composition photoréticulable, et
B) exposition de l'ongle ou du faux-ongle revêtu obtenu à l'issue de l'étape A) à un rayonnement UV ou de lumière visible.

**16.** Procédé de revêtement des ongles et/ou des faux-ongles, en particulier de maquillage et/ou de soin des ongles et/ou des faux-ongles, comprenant au moins les étapes suivantes :

A) application, sur un ongle ou un faux-ongle, d'une première composition selon l'une quelconque des revendications 1 à 14, par laquelle on dépose un premier revêtement constitué d'au moins une couche de ladite composition photoréticulable, ce premier revêtement étant appliqué directement au contact de l'ongle ou du faux-ongle, et
B) exposition, de l'ongle ou du faux-ongle revêtu obtenu à l'issue de l'étape A) à un rayonnement UV ou de lumière visible,
C) application, sur le premier revêtement issu des étapes A) et B), d'une deuxième composition, distincte de la première composition, par laquelle on dépose un deuxième revêtement constitué d'au moins une couche de ladite deuxième composition,
D) exposition de l'ongle ou du faux-ongle revêtu obtenu à l'issue de l'étape C) à un rayonnement UV ou de lumière visible.

**17.** Procédé de revêtement des ongles et/ou des faux-ongles, en particulier de maquillage et/ou de soin des ongles et/ou des faux-ongles, comprend au moins les étapes suivantes :

A) application, sur un ongle ou un faux-ongle, d'une première composition selon l'une quelconque des revendications 1 à 14, par laquelle on dépose un premier revêtement constitué d'au moins une couche de ladite composition photoréticulable, ce premier revêtement étant appliqué directement au contact de l'ongle ou du faux-ongle, et
B) exposition de l'ongle ou du faux-ongle revêtu obtenu à l'issue de l'étape A) à un rayonnement UV ou de lumière visible,
C) application, sur le premier revêtement issu des étapes A) et B), d'une deuxième composition, distincte de la première composition, par laquelle on dépose un deuxième revêtement constitué d'au moins une couche de ladite deuxième composition,
D) exposition, de l'ongle ou du faux-ongle revêtu obtenu à l'issue de l'étape C) à un rayonnement UV ou de lumière visible,

E) application sur le deuxième revêtement issu des étapes C) et D), d'une troisième composition, distincte de la première composition et de la deuxième composition, par laquelle on dépose un troisième revêtement constitué d'au moins une couche de ladite troisième composition,
F) exposition de l'ongle ou du faux-ongle revêtu obtenu à l'issue de l'étape E) à un rayonnement UV ou de lumière visible.

**18.** Procédé de revêtement des ongles et/ou des faux-ongles, en particulier de maquillage et/ou de soin des ongles et/ou des faux-ongles, comprenant au moins les étapes suivantes :

A) application, sur un ongle ou un faux-ongle, d'une première composition selon l'une quelconque des revendications 1 à 14, par laquelle on dépose un premier revêtement constitué d'au moins une couche de ladite composition photoréticulable,
B) exposition de l'ongle ou du faux-ongle revêtu obtenu à l'issue de l'étape A) à un rayonnement UV ou de lumière visible,
C) application, sur le premier revêtement issu des étapes A) et B), d'une deuxième composition, distincte de la première composition, par laquelle on dépose un deuxième revêtement constitué d'au moins une couche de ladite deuxième composition, la deuxième composition comprenant :

- au moins un composé uréthane (méth)acrylate répondant à la formule (XIII) suivante :

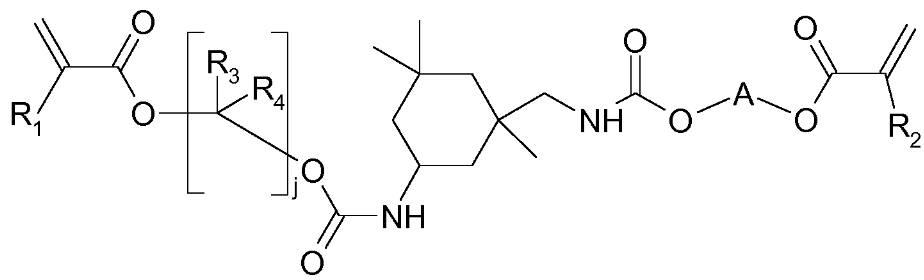

Formule (XIII)

Formule XIII dans laquelle :

- $R_1$, $R_2$, $R_3$, et $R_4$, identiques ou différents, représentent un atome d'hydrogène ou une chaîne alkyle en $C_1$-$C_{10}$, de préférence un atome d'hydrogène ou un groupe méthyle,
- j est compris entre 1 et 10, de préférence égal à 2,
- A représente un groupe alkyle en $C_1$-$C_{10}$, ou un polyuréthane comprenant de 2 à 20 motifs carbamates.

- au moins un composé photoréticulable b) répondant à la formule (XI) suivante :

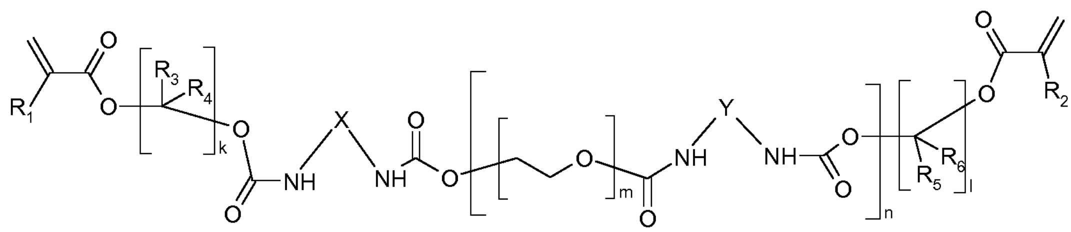

Formule (XI)

Formule (XI) dans laquelle :

- $R_1$, $R_2$, $R_3$, $R_4$, $R_5$, et $R_6$, identiques ou différents, représentent un atome d'hydrogène ou une chaîne alkyle en $C_1$-$C_{10}$, de préférence un atome d'hydrogène ou un groupe méthyle,
- k et l, identiques ou différents, sont compris entre 1 et 10, de préférence sont égaux à 2,
- m est compris entre 1 et 100, de préférence compris entre 5 et 50, de préférence compris entre

environ 8 et 10,
- n est compris entre 1 et 10, de préférence égal à 1,
- X et Y, identiques ou différents, représentent un groupe cycloalkyle ou alkyle en $C_1$-$C_{20}$,

- au moins un photoamorceur, de préférence choisi dans le groupe constitué des $\alpha$-hydroxycétones, des $\alpha$-aminocétones, des cétones aromatiques de préférence associées à un composé donneur d'hydrogène, des $\alpha$-dicétones aromatiques et des oxydes d'acylphosphine, et de leurs mélanges, avantageusement dans le groupe constitué des oxydes d'acylphosphine,
- de préférence au moins un monomère (méth)acrylate monofonctionnel, de préférence du tetrahydrofurfuryl méthacrylate (THFMA),
- éventuellement au moins un agent de coloration, en particulier au moins un pigment,

D) exposition de l'ongle ou du faux-ongle revêtu obtenu à l'issue de l'étape C) à un rayonnement UV ou de lumière visible,
E) éventuellement application, sur le deuxième revêtement issu des étapes C) et D), d'une troisième composition, par laquelle on dépose un troisième revêtement constitué d'au moins une couche de ladite troisième composition, la troisième composition comprenant :

- au moins un composé uréthane (méth)acrylate répondant à la formule (XIII) suivante :

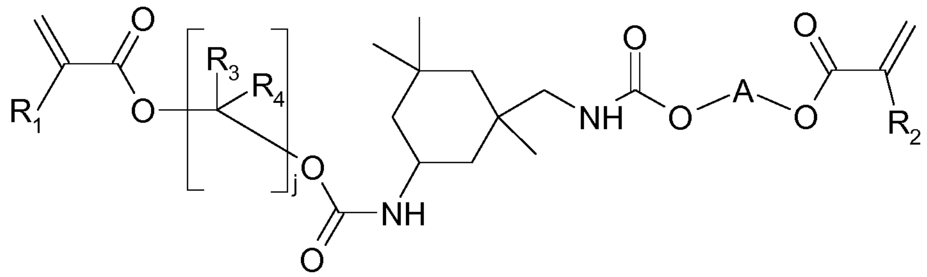

Formule (XIII)

Formule (XIII) dans laquelle :

- $R_1$, $R_2$, $R_3$, et $R_4$, identiques ou différents, représentent un atome d'hydrogène ou une chaîne alkyle en $C_1$-$C_{10}$, de préférence un atome d'hydrogène ou un groupe méthyle,
- j est compris entre 1 et 10, de préférence égal à 2,
- A représente un groupe alkyle en $C_1$-$C_{10}$, ou un polyuréthane comprenant de 2 à 20 motifs carbamates.

- au moins un composé photoréticulable b) répondant à la formule (XI) suivante :

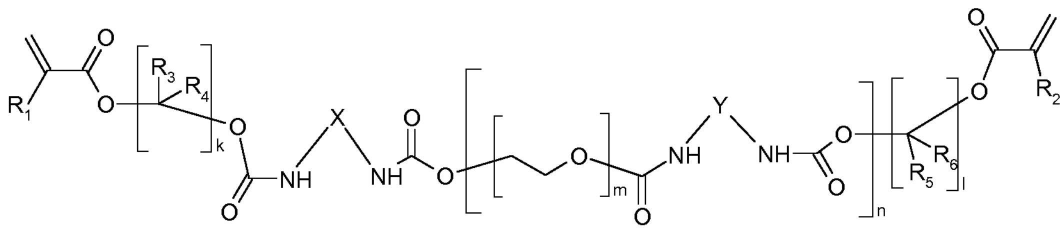

Formule (XI)

Formule (XI) dans laquelle :

- $R_1$, $R_2$, $R_3$, $R_4$, $R_5$, et $R_6$, identiques ou différents, représentent un atome d'hydrogène ou une chaîne alkyle en $C_1$-$C_{10}$, de préférence un atome d'hydrogène ou un groupe méthyle,
- avec k et l, identiques ou différents, compris entre 1 et 10, de préférence égaux à 2,
- avec m compris entre 1 et 100, de préférence compris entre 5 et 50, de préférence compris entre environ 8 et 10,

- avec n compris entre 1 et 10, de préférence égal à 1,
- avec X et Y, identiques ou différents, représentant un groupe cycloalkyle ou alkyle en $C_1$-$C_{20}$,

- au moins un photoamorceur, de préférence choisi dans le groupe constitué des α-hydroxycétones, des α-aminocétones, des cétones aromatiques de préférence associées à un composé donneur d'hydrogène, des α-dicétones aromatiques et des oxydes d'acylphosphine, et de leurs mélanges, avantageusement dans le groupe constitué des oxydes d'acylphosphine,
- de préférence au moins un monomère (méth)acrylate monofonctionnel, de préférence du tetrahydrofurfuryl méthacrylate (THFMA),

F) exposition de l'ongle ou du faux-ongle revêtu obtenu à l'issue de l'étape E) à un rayonnement UV ou de lumière visible.

**19.** Procédé de revêtement des ongles et/ou des faux-ongles selon la revendication 17 ou 18, dans lequel, lorsqu'un troisième revêtement d'une troisième composition est appliqué, la deuxième composition appliquée en tant que deuxième revêtement comprend au moins un agent de coloration.

**20.** Procédé de revêtement des ongles et/ou des faux-ongles selon la revendication 17, 18 ou 19, dans lequel les étapes C) et D) sont répétées au total deux fois, la deuxième composition étant appliquée une première fois et exposée à un rayonnement UV ou de lumière visible, puis étant appliquée une deuxième fois et exposée à un rayonnement UV ou de lumière visible, de préférences les étapes A) et B), de même que E) et F) n'étant respectivement mises en oeuvre qu'une seule fois.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CA 1306954
- US 5456905 A
- US 7375144 B
- FR 2823105
- US 20110081306 A
- US 20110082228 A
- US 20110274633 A
- US 20120083547 A
- US 20130263875 A

### Non-patent literature cited in the description

- **C.M. HANSEN.** The three dimensional solubility parameters. *J. Paint Technol.,* 1967, vol. 39, 105
- **G. LI BASSI.** Les photoinitiateurs dans la réticulation des revêtements'' [''Photoinitiators in the crosslinking of coatings. *Double Liaison - Chimie des Peintures,* November 1985, 34-41
- **HENRI STRUB.** Applications industrielles de la polymerisation photoinduite'' [''Industrial applications of photoinduced polymerisation. *L'Actualité Chimique,* February 2000, 5-13
- **MARC, J.M. ABADIE.** Photopolymères: considerations théoriques et réaction de prise'' [''Photopolymers: theoretical considerations and setting reaction. *Double Liaison - Chimie des Peintures,* 1992, 28-34